# EUROPEAN PATENT SPECIFICATION

(11) **EP 3 771 423 B1**
(45) Date of publication and mention of the grant of the patent: **07.10.2026**
(21) Application number: 20189207.2
(22) Date of filing: 03.08.2020
(51) Int. Cl.: A61B 5/145

(54) **INSERTION DEVICE FOR A BIOSENSOR**
EINFÜHRVORRICHTUNG FÜR EINEN BIOSENSOR
DISPOSITIF D'INSERTION D'UN BIOCAPTEUR

(30) Priority: 02.08.2019 US 201962882140 P; 10.01.2020 TW 109100966
(43) Date of publication of application: 03.02.2021
(73) Proprietor: Bionime Corporation, Taichung City 40242 (TW)
(72) Inventor: HUANG, Chun-Mu, 40242 Taichung City (TW); CHEN, Chieh-Hsing, 40242 Taichung City (TW); LEE, Chen-Hao, 40242 Taichung City (TW); CHANG, Kuan-Lin, 40242 Taichung City (TW)
(74) Representative: Page White Farrer

(56) References cited:
- WO-A1-2018/222012
- US-A1- 2005 101 912
- US-A1- 2017 188 912
- US-A1- 2018 317 820

## Description

The disclosure relates to an insertion device, and more particularly to an insertion device for a biosensor.

A conventional insertion device disclosed in U.S. Patent No. 10413183 is for inserting a biosensor into a host, and includes a plunger, and a piercing member to which the sensor assembly is mounted.

The piercing member is inserted into the host upon depression of the plunger, but is not separated from the host until the plunger is released. Accordingly, the conventional insertion device is operated via a two-action operation. When an operator fails to promptly release the plunger, the piercing member remains to be inserted into the host and may cause discomfort of the host.

WO 2018/222012 A1 presents an insertion device comprising a lower casing which abuts against a lower surrounding wall of an upper casing. A locking piece prevents depression of the press bottom, and is pulled laterally to unlock the press bottom to allow the insertion device to be engaged.

US 2018/317820 A1 presents an insertion device comprising a container and an applicator containing a sensor. **In** use, by pushing an applicator assembly of the applicator toward the container, a needle assembly in the container is coupled to the applicator assembly and can be applied to an application site.

US 2017/188912 A1 presents an insertion device comprising a first portion and a second portion, where the first portion is pushed towards the second portion for insertion of a needle, and where the lower portion abuts against a lower surrounding wall of the upper portion.

US 2005/101912 A1 presents an insertion device where a lower casing is coupled to an upper casing and abuts against a lower end of the upper casing. In operation the lower casing is removed from the upper casing before performing an insertion operation.

Therefore, an object of the disclosure is to provide an insertion device that can alleviate the drawback of the prior art.

According to the disclosure, there is provided an insertion device according to claim 1.

Other features and advantages of the disclosure will become apparent in the following detailed description of the embodiments with reference to the accompanying drawings, of which:
Figure 1 is an exploded perspective view illustrating a first embodiment of the insertion device according to the disclosure;
Figure 2 is another exploded perspective view illustrating the first embodiment;
Figure 3 is a sectional view illustrating of first embodiment, illustrating a sensor of a container in a state to be implanted;
Figure 4 is a sectional view taken along line IV-IV in Figure 3;
Figure 5 is a sectional view illustrating a lower casing of the first embodiment uncovering a base;
Figure 6 is a sectional view taken along line VI-VI in Figure 5;
Figure 7 is a sectional view illustrating an upper casing of the first embodiment being depressed;
Figure 8 is a sectional view taken along line VIII-VIII in Figure 7;
Figure 9 is a sectional view illustrating an insertion seat of the first embodiment being moved toward a host;
Figure 10 is a sectional view taken along line X-X in Figure 9;
Figure 11 is a sectional view illustrating a retraction seat of the first embodiment being moved away from the host;
Figure 12 is a sectional view taken along line XII-XII in Figure 11;
Figure 13 is a sectional view illustrating an auxiliary insertion seat of the first embodiment being separated from a sensor assembly;
Figure 14 is a sectional view taken along line XIV-XIV in Figure 13;
Figure 15 is a sectional view illustrating the lower casing being re-coupled to the upper casing;
Figure 16 is a sectional view taken along line XVI-XVI in Figure 15;
Figure 17 is a perspective view illustrating a modification of the insertion device;
Figure 18 is a sectional view of the modification shown in Fig. 17, illustrating a stage of being prepared for the implanting;
Figure 19 is a sectional view taken along line XIX-XIX in Figure 18;
Figure 20 is another sectional view of the modification, illustrating an instance during the implanting;
Figure 21 is a sectional view taken along line XXI-XXI in Figure 20;
Figure 22 is a schematic views illustrating a second embodiment of the insertion device according to the disclosure being assembled;
Figure 23 is a schematic view illustrating a lower casing of the second embodiment uncovering a base;
Figure 24 is a schematic view illustrating operation of an insertion module;
Figure 25 is a schematic view illustrating the lower casing covering the base;
Figure 26 is a schematic view illustrating the lower casing tearing a release layer via a tearing element when the lower casing uncovers the base;
Figure 27 is a flow chart of an insertion operation of the insertion device according to the disclosure;
Figure 28 is a sectional view illustrating a sensor assembly being mounted onto a base;
Figure 29 is a partly sectional view illustrating the sensor assembly being connected to an auxiliary insertion seat;
Figure 30 is another exploded perspective view of the insertion device;
Figure 31 is a sectional view illustrating a third embodiment of the insertion device according to the disclosure;
Figure 32 is an enlarged sectional view of the third embodiment; and
Figure 33 is a sectional view illustrating a fourth embodiment of the insertion device according to the disclosure.

Before the disclosure is described in greater detail, it should be noted that where considered appropriate, reference numerals or terminal portions of reference numerals have been repeated among the figures to indicate corresponding or analogous elements, which may optionally have similar characteristics.

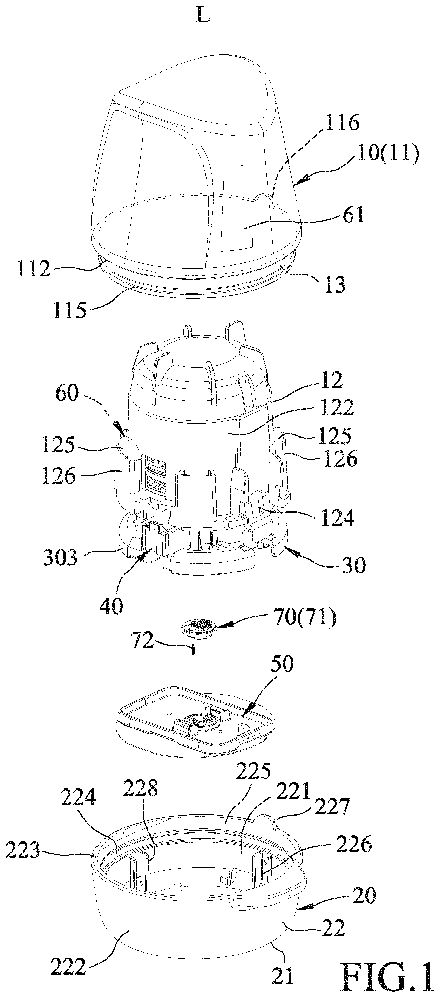

Referring to Figures 1 to 4, a first embodiment of the insertion device according to the disclosure is for inserting a sensor into a host. The insertion device may be in the form of a canister (e.g., a desiccant canister), but is not limited to such. The insertion device includes an upper casing 10 and an insertion module 30.

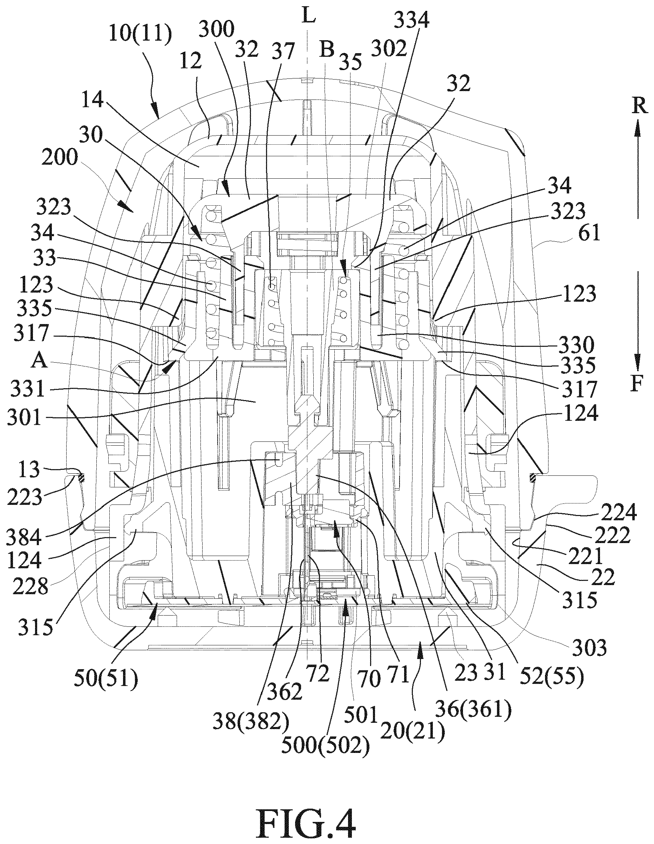

The upper casing 10 has an accommodating space 14 (see Figure 4), and a pair of casing engaging structures 124 (see Figure 4).

The insertion module 30 is disposed in the accommodating space 14, and includes a main body assembly 300, an insertion seat 33, a first elastic member 34, a retraction seat 35, a second elastic member 37, a base 50 and a sensor assembly 70.

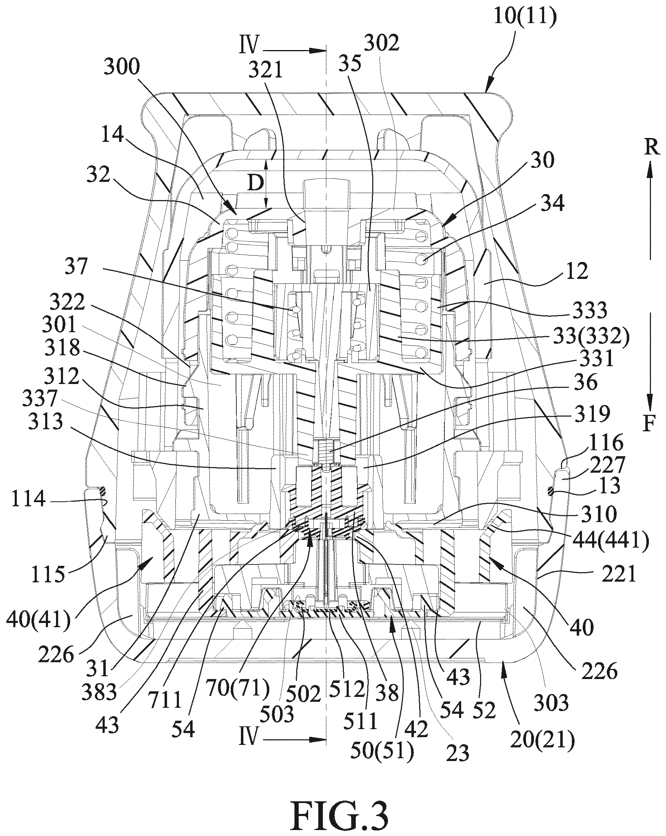

With particular reference to Figures 3 and 4, the main body assembly 300 includes an upper portion 302, and a lower portion 303 that cooperates with the upper portion 302 to define a displacement space 301 therebetween. The lower portion 303 of the main body assembly 300 has a pair of body engaging structures 315 that abut against inner sides of the casing engaging structures 124 of the upper casing 10, and that are able to respectively engage the casing engaging structures 124 of the upper casing 10 when the upper casing 10 is depressed downwardly.

The insertion seat 33 is removably positioned in the upper portion 302 of the main body assembly 300, and is able to move in the displacement space 301 between the upper portion 302 and the lower portion 303 along an axial line (L).

The upper casing 10 has a pair of urging portions 123 (see Figure 4). The lower portion 303 of the main body assembly 300 has a pair of stopping portions 317 (see Figure 4). The insertion seat 33 has a pair of buckle portions 335 (see Figure 4) that respectively and separably abut against the stopping portions 317 of the lower portion 303 of the main body assembly 300 so as to position the insertion seat 33 relative to the main body assembly 300, and that are able to be respectively pushed by the urging portions 123 of the upper casing 10 to be respectively separated from the stopping portions 317 of the lower portion 303 of the main body assembly 300. The buckle portions 335 of the insertion seat 33 respectively and separably abut the stopping portions 317 of the lower portion 303 of the main body assembly 300 so as to form an insertion limiting structure (A) (see Figure 4).

The insertion seat 33 further has at least one limiting groove 330 (see Figure 4). The upper portion 302 of the main body assembly 300 has at least one limiting member 323 (see Figure 4) that removably engages with the limiting groove 330 of the insertion seat 33. The insertion seat 33 further has at least one retraction positioning portion 334 that separably abuts against the limiting member 323 of the upper portion 302 of the main body assembly 300.

The first elastic member 34 has two opposite ends respectively abutting against the insertion seat 33 and the upper portion 302 of the main body assembly 300. In this embodiment, the first elastic member 34 may be configured as a pre-compressed spring.

The retraction seat 35 is mounted with an insertion needle 36, and is removably positioned relative to the insertion seat 33. Specifically, the retraction positioning portion 334 of the insertion seat 33 is removably limited by the limiting member 323 of the upper portion 302 of the main body assembly 300, so as to form a retraction limiting structure (B) (see Figure 4) that positions the retraction seat 35 relative to the insertion seat 33.

The second elastic member 37 has two opposite ends respectively abutting against the insertion seat 33 and the retraction seat 35. In this embodiment, the second elastic member 37 may be configured as a pre-compressed spring.

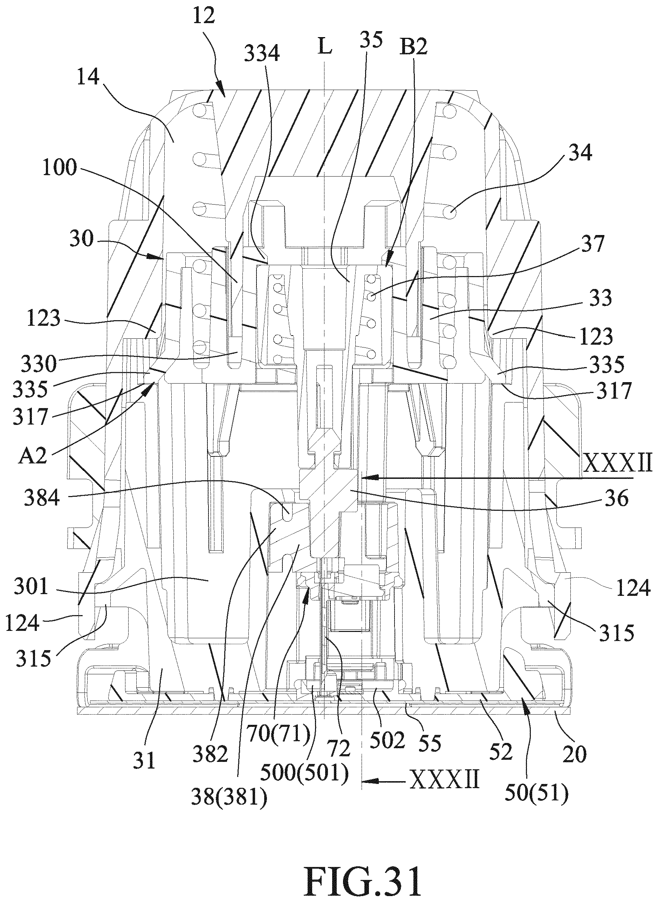

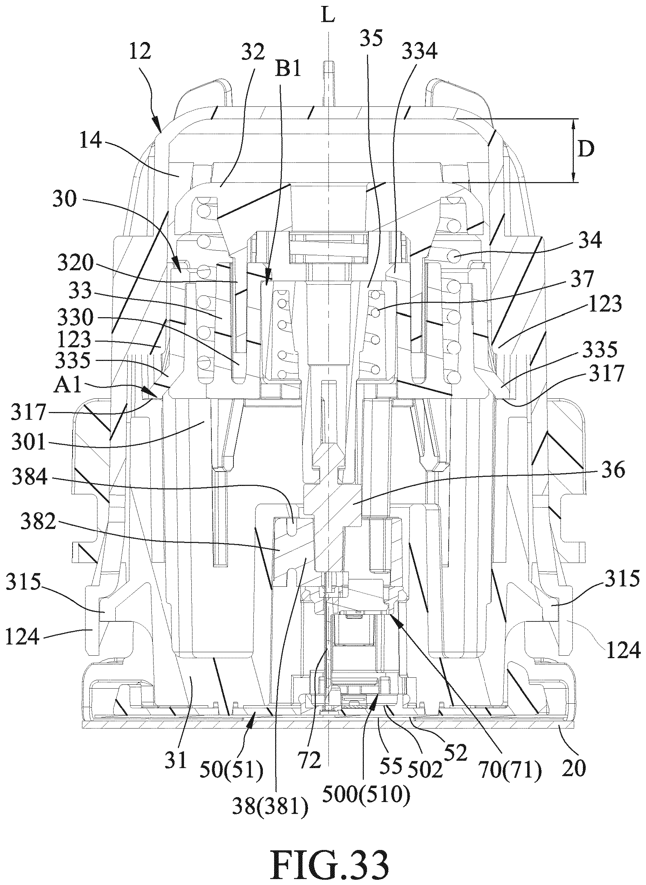

The base 50 is separably positioned relative to the lower portion 303 of the main body assembly 300, and includes a base seat 51, and an adhesive pad 52 that is fixedly connected to the base seat 51. In one embodiment, the base 50 may further include a release layer 55 (see Figures 31 and 33) that is separably adhered to the adhesive pad 52.

The insertion module 30 further includes an auxiliary insertion seat 38 that is separably mounted to the insertion needle 36. The sensor assembly 70 is separably mounted to the auxiliary insertion seat 38. The auxiliary insertion seat 38 has a base portion 381, three angularly spaced-apart wing portions 382 each extending radially outwardly from the base portion 381, and a plurality of coupling portions 383 (see Figure 3) that protrude from a bottom surface of the base portion 381. Each of the wing portions 382 has a plurality of notches 384, such that the wing portion 382 is flexible in a radial direction of the auxiliary insertion seat 38.

Referring to Figures 3 and 4, after first embodiment of the insertion device is assembled, the upper portion 302 and the lower portion 303 of the main body assembly 300 are interconnected, a top portion of the upper casing 10 and a top portion of the upper portion 302 of the main body assembly 300 are spaced apart from each other by a distance (D) (see Figure 3), the buckle portions 335 of the insertion seat 33 respectively abut against the stopping portions 317 of the lower portion 303 of the main body assembly 300 so as to position the insertion seat 33 at a pre-insertion position, the first elastic member 34 is pre-compressed between the insertion seat 33 and the upper portion 302 of the main body assembly 300 to generate a restoring force, the second elastic member 37 is pre-compressed between the retraction seat 35 and the insertion seat 33 to generate a restoring force, the retraction seat 35 is at a pre-insertion position, the coupling portions 383 of the auxiliary insertion seat 38 is fitted into the coupling portion 711 of the sensing seat 71 so that the sensing seat 71 and the auxiliary insertion seat 38 are interconnected, the auxiliary insertion seat 38 is connected to a needle seat 361 of the insertion needle 36 so that the insertion needle 36 is retained in the lower portion 303 of the main body assembly 300 and is shielded by the base 50, and the base 50 is positioned relative to the lower portion 303 of the main body assembly 300.

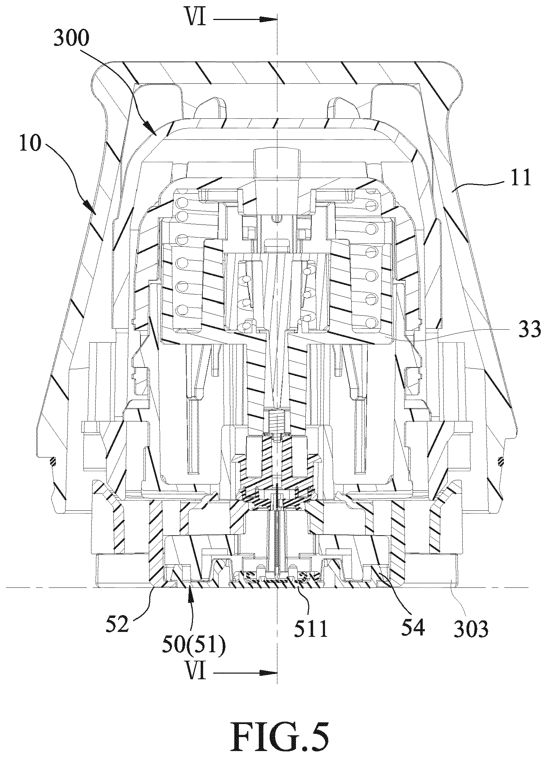

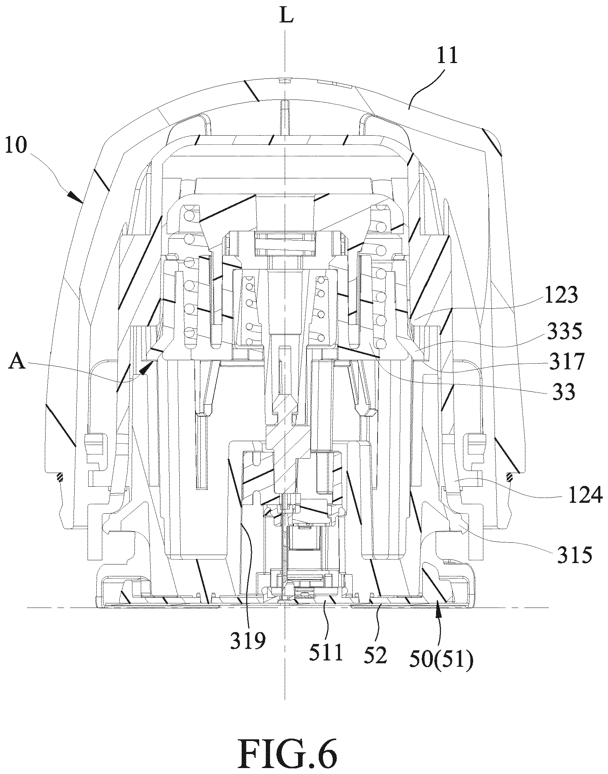

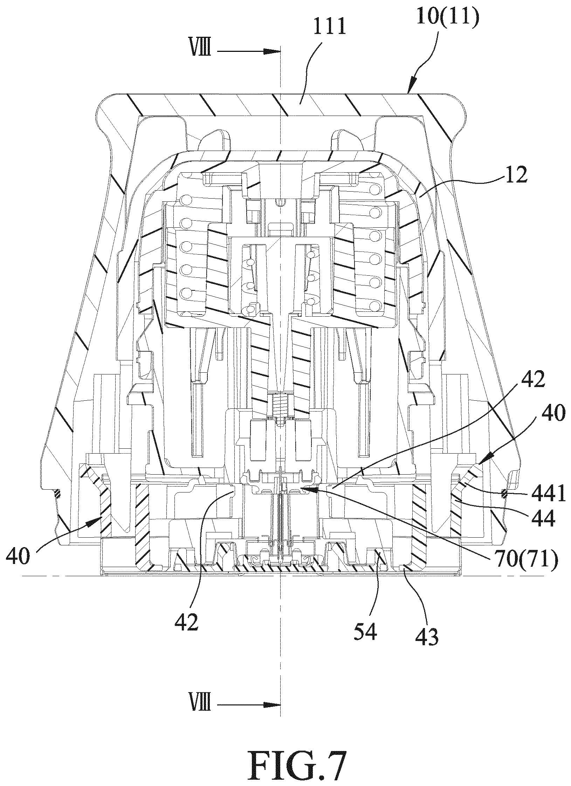

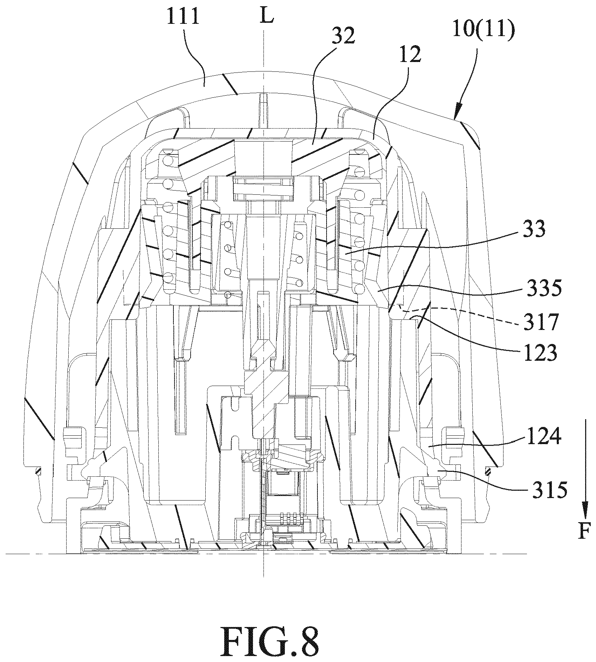

The insertion operation performed by the first embodiment (to insert the sensor 72 into the host) is illustrated as follows:
Referring to Figures 5 and 6, the adhesive pad 52 is adhered to a skin surface of the host. Referring to Figures 7 and 8, when the upper casing 10 is not depressed, the insertion limiting structure (A) that is formed between the buckle portions 335 of the insertion seat 33 and the stopping portions 317 of the lower portion 303 of the main body assembly 300 maintains the insertion seat 33 at the pre-insertion position. When the upper casing 10 is depressed toward the skin surface, the buckle portions 335 of the insertion seat 33 are respectively pushed by the urging portions 123 of the upper casing 10 to be deformed inwardly and to be respectively separated from the stopping portions 317 of the lower portion 303 of the main body assembly 300, so that the insertion limiting structure (A) is collapsed. At the same time, the body engaging structures 315 of the main body assembly 300 respectively engage the casing engaging structure 124 of the upper casing 10, so that the upper casing 10 is positioned relative to the main body assembly 300. After the cover body 12 being pressed by the user, the actuating portions 123 (i.e., the urging portions 123) will be stuck underneath the main body 31, and thus the cover body 12 cannot be moved upward while the user does not feel any vibration nor noise

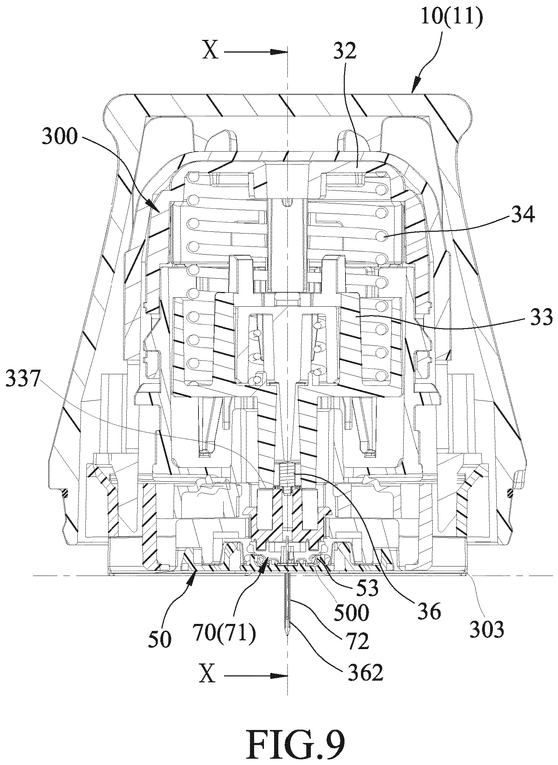

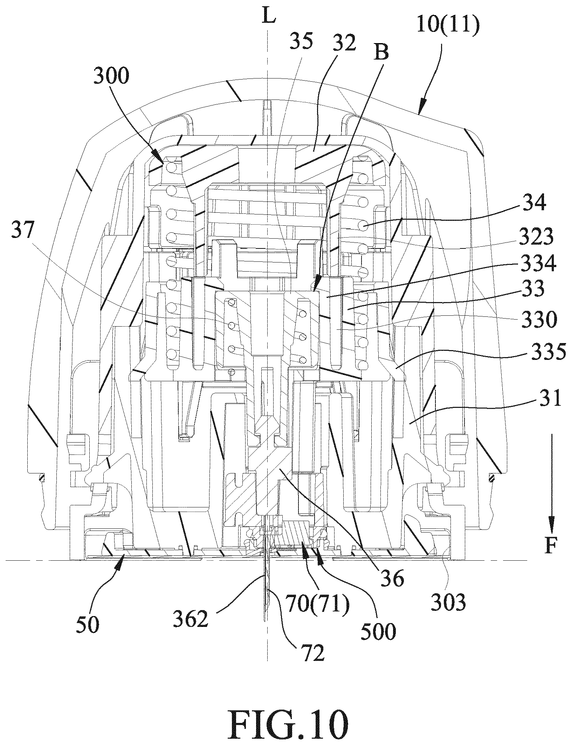

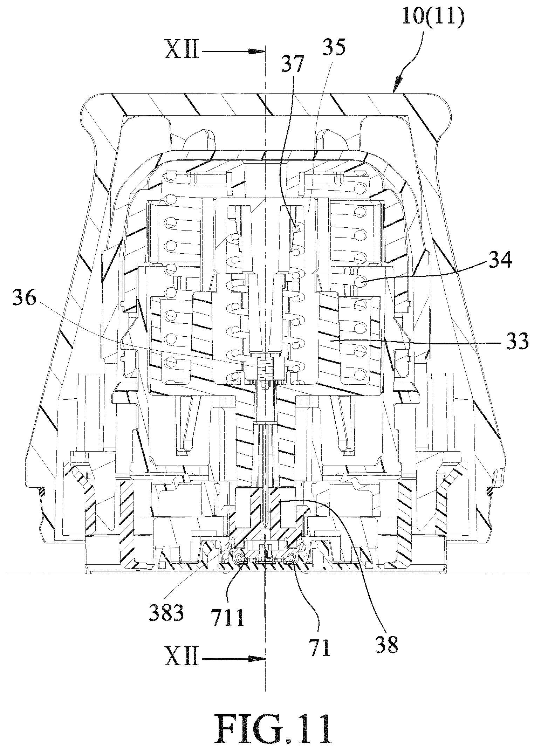

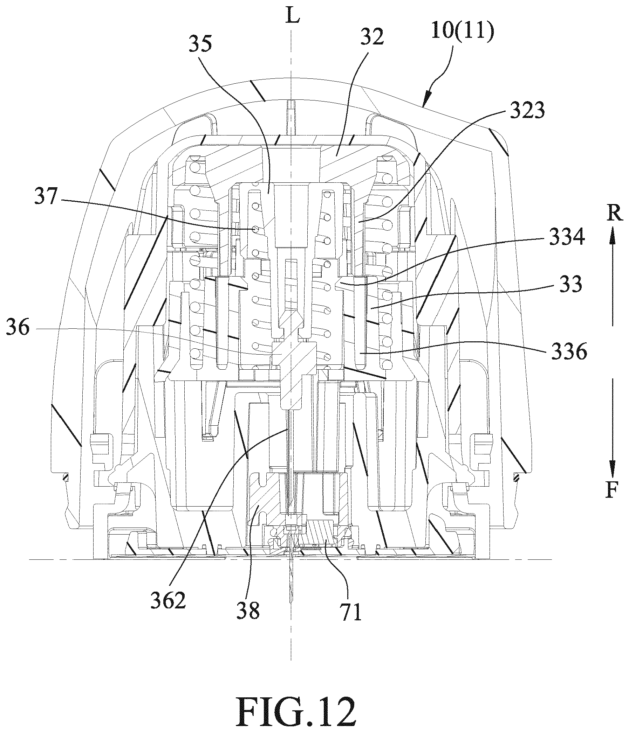

Referring to Figures 9 and 10, after the insertion limiting structure (A) is collapsed, the restoring force of the first elastic member 34 is permitted to be released, and moves the insertion seat 33 to an insertion position to implement automatic-insertion, such that the sensor assembly 70 is moved by the insertion seat 33 to a post-insertion position, that a portion of the sensor 72 is inserted underneath the skin surface, and that the sensing seat 71 is positioned onto the mounting portion 500 of the base 50. After the sensor 72 is inserted underneath the skin surface, the limiting member 323 of the upper portion 302 of the main body assembly 300 is separated from the limiting groove 330 and the retraction positioning portion 334 of the insertion seat 33, so that the retraction positioning portion 334 is permitted to be deformed outwardly to collapse the retraction limiting structure (B). As such, the restoring force of the second elastic member 37 is permitted to be released, and drives the retraction seat 35 to move past the retraction positioning portion 334 of the insertion seat 33 away from the skin surface, such that the insertion needle 36 is separated from the auxiliary insertion seat 38 and is retracted into the insertion seat 33 to hide a needle 362 thereof and to implement automatic-retraction (see Figures 11 and 12). At this time, the coupling portions 383 of the auxiliary insertion seat 38 is maintained to be fitted into the coupling portion 711 of the sensing seat 71 so that the auxiliary insertion seat 38 is connected to the sensing seat 71 of the sensor assembly 70.

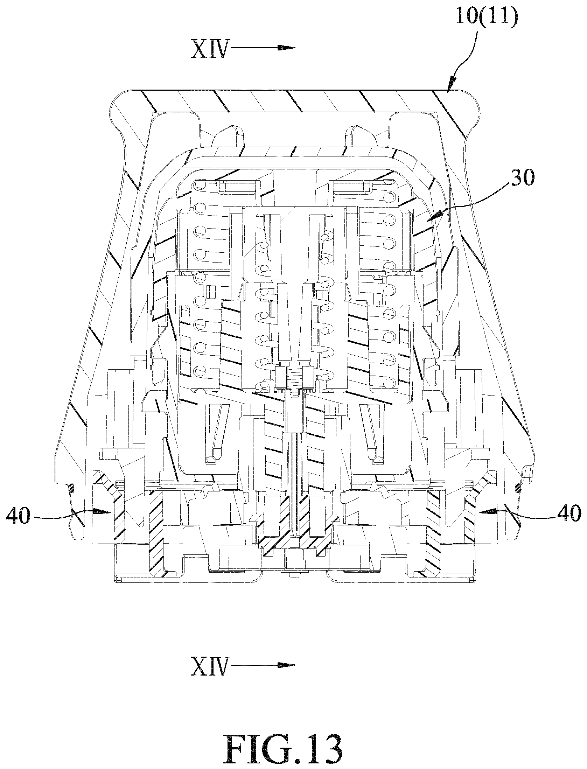

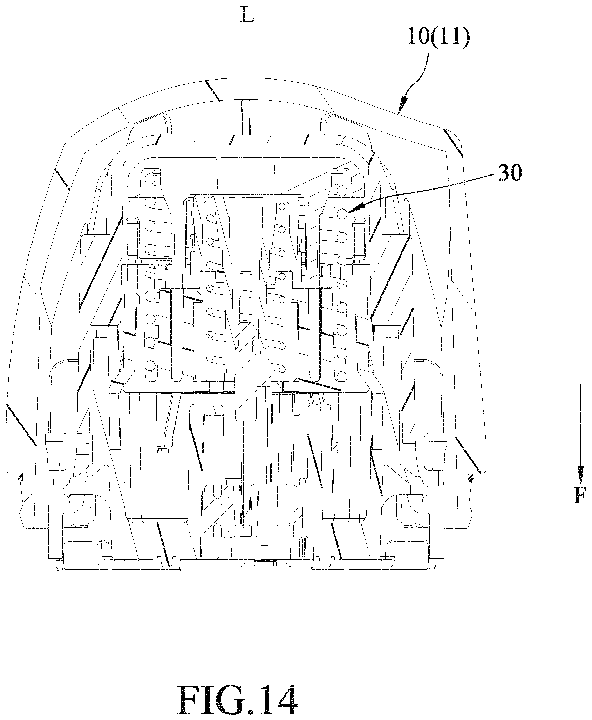

Afterward, the upper casing 10 and the insertion module 30 are separated from the base 50 and the skin surface of the host (the auxiliary insertion seat 38 is separated from the sensing seat 71 of the sensor assembly 70), so as to finish the insertion operation of the first embodiment of the insertion device including the automatic-insertion and automatic-retraction operations (see Figures 13, 14 and 28).

Accordingly, the first embodiment of the insertion device according to the disclosure is operated via a single-action operation. When the upper casing 10 is depressed, the insertion seat 33 is driven by the compressed first elastic member 34 to move toward the skin surface so as to perform the automatic-insertion operation and to separate the limiting structures (i.e., the limiting groove 330 and the limiting member 323) between the insertion seat 33 and the upper portion 302 of the main body assembly 300. After the automatic-insertion operation is done, the upper casing 10 is positioned relative to the main body assembly 300 without rebounding, and the limiting structures between the insertion seat 33 and the upper portion 302 are separated from each other, so that the retraction seat 35 is driven by the pre-compressed second elastic member 37 to perform the automatic-retraction operation. In this embodiment, a time needed for performing the automatic-insertion operation and the automatic-retraction operation is no more than 100 milliseconds. In other words, during the insertion operation of the first embodiment, the duration for which the insertion needle 36 is inserted into the host is no more than 100 milliseconds. In other embodiments, the duration may be no more than 50 milliseconds, no more than 8 milliseconds, no more than 6 milliseconds, no more than 4 milliseconds or no more than 2 milliseconds.

Referring back to Figures 1 to 4, a modification of the first embodiment of the insertion device according to the disclosure is for inserting a sensor into a host. The modification may also be in the form of a canister (e.g., a desiccant canister), but is not limited to such.

The modification of the insertion device includes an upper casing 10, a lower casing 20, an insertion module 30, a base 50 and a desiccant 60.

The upper casing 10 includes an outer shell 11, and a cover body 12 that is disposed in the outer shell 11. The cover body 12 has at least one casing engaging structure 124 at a lower portion thereof. The outer casing 11 has an annular protruding portion 115 and an upper positioning portion 116.

The lower casing 20 is separably and airtightly connected to the upper casing 10 so as to form an airtight space 200 (see Figure 4) therebetween. The desiccant 60 is disposed in the airtight space 200.

In this modification, the upper casing 10 and the lower casing 20 are interconnected in a hard-interference manner, but are not limited to such. The lower casing 20 has a top edge 233. The top edge 233 of the lower casing 20 is provided with a lower positioning portion 227 that corresponds in position and shape to the upper positioning portion 116 of the upper casing 10. The lower casing 20 further has at least one abutment portion 228 at an inner surrounding surface thereof that abuts against an outer side of the casing engaging structure 124 of the upper casing 10, and an annular groove 224 at the inner surrounding surface thereof that permits the annular protruding portion 115 of the upper casing 10 to engage therewith so as to interconnect the upper casing 10 and the lower casing 20.

In this modification, the insertion device may further include a sealing ring 13 (see Figures 3 and 4) that is disposed between the annular protruding portion 115 of the upper casing 10 and the annular groove 224 of the lower casing 20 for enhancing air-tightness of the airtight space 200 defined between the upper casing 10 and the lower casing 20.

The insertion module 30 is disposed in the upper casing 10, and is able to be moved by the upper casing 10. The insertion module 30 includes a main body assembly 300. The main body assembly 300 includes a main body 31, and a main cover 32 that is connected to the main body 31 and that cooperates with the main body 31 to define a displacement space 301 therebetween. The main body assembly 300 further has at least one body engaging structure 315 that abuts against an inner side of the casing engaging structure 124 of the upper casing 10. The body engaging structure 315 engages with the casing engaging structure 124 of the upper casing 10 when the upper casing 10 is depressed.

In this modification, the body engaging structure 315 of the main body assembly 300 is disposed on the main body 31 of the main body assembly 300, and the body engaging structure 315 abuts against the inner side of the casing engaging structure 124 of the cover body 12 of the upper casing 10. When the lower casing 20 engages the upper casing 10, the abutment portion 228 of the lower casing 20 limits movement of the casing engaging structure 124 of the cover body 12 of the upper casing 10, such that the upper casing 10 cannot move downwardly so as to prevent unintentional insertion operation of the insertion device due to unintentionally applying force to the insertion device.

The insertion module 30 further includes an insertion seat 33 that is disposed in the displacement space 301, a first elastic member 34 that has two opposite ends respectively abutting against the insertion seat 33 and the main cover 32 for moving the insertion seat 33 in an insertion direction (F), a retraction seat 35 that is disposed in the insertion seat 33, an insertion needle 36 that is connected to the retraction seat 35, a second elastic member 37 that has two opposite ends respectively abutting against the insertion seat 33 and the retraction seat 35 for moving the retraction seat 35 in a retraction direction (R) opposite to the insertion direction (F), an auxiliary insertion seat 38 that is separably connected to the insertion needle 36, and a sensor assembly 70 that is separably connected to the auxiliary insertion seat 38. The sensor assembly 70 includes a sensing seat 71, and a sensor 72 that is mounted to the sensing seat 71 and that is separably coupled to the insertion needle 36.

In this modification, the first elastic member 34 and the second elastic 37 are configured as pre-compressed springs.

The cover body 12 of the upper casing 10 has a pair of urging portions 123 at an inner surrounding surface thereof. The insertion seat 33 has a pair of buckle portions 335 (see Figure 4) that respectively and separably abut against two stopping portions 317 of the main body 31, and that are able to be respectively pushed by the urging portions 123 of the cover body 12 to be respectively separated from the stopping portions 317 of the main body 31. The buckle portions 335 of the insertion seat 33 respectively and separably abut the stopping portions 317 of the main body 31 so as to form an insertion limiting structure (A) between the insertion seat 33 and the main body 31.

The insertion seat 33 further has at least one limiting groove 330. The main cover 32 has at least one limiting member 323 that removably engages with the limiting groove 330 of the insertion seat 33. The insertion seat 33 further has at least one retraction positioning portion 334 that separably abuts against the limiting member 323 of the main cover 32 and that limits movements of the retraction seat 35. The retraction positioning portion 334 of the insertion seat 33 is limited by the limiting member 323 of the main cover 32, so as to form a retraction limiting structure (B) among the retraction seat 35, the insertion seat 33 and the main cover 32 of the main body assembly 300.

In this embodiment, the sensor assembly 70 is to be separably mounted to the base 50, and includes a sensing seat 71, and a sensor 72 that is mounted to the sensing seat 71 and that is separably coupled to the insertion needle 36. The sensing seat 71 has a coupling portion 711 (see Figure 3).

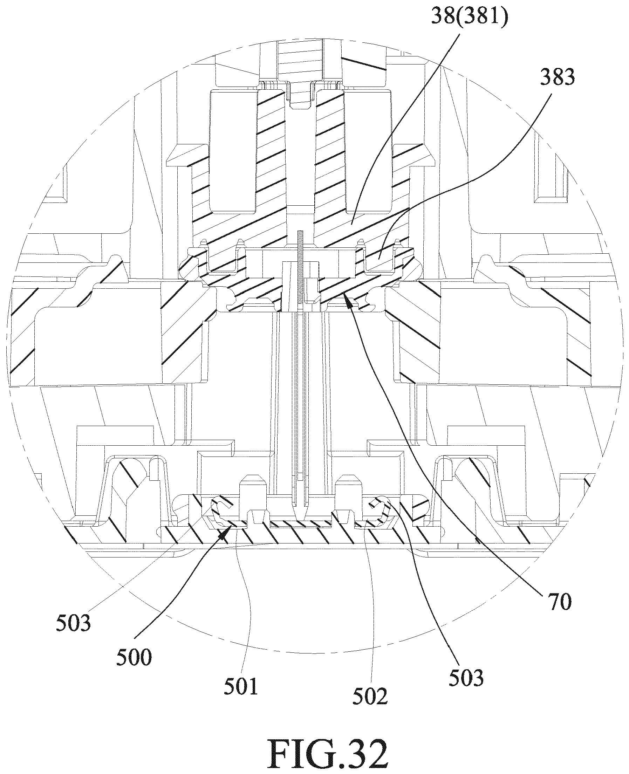

The base 50 is separably positioned relative to the main body 32 of the main body assembly 300, and permits the sensor assembly 70 to be mounted thereon after the sensor assembly 70 is separated from the auxiliary insertion seat 38. The base 50 includes a base seat 51, an adhesive pad 52 that is fixedly connected to the base seat 51, and two base hook structures 54 (see Figure 3). In one embodiment, the base 50 may further include a release layer 55 (see Figures 31 and 33) that is separably adhered to the adhesive pad 52. The base 50 further has a mounting portion 500 (see Figure 4) for the sensor assembly 70 to be separably mounted thereto. In this modification, the mounting portion 500 of the base 50 includes a recess 501 that permits the sensor assembly 70 to be press-fitted thereinto, or may include an adhesive layer 502 (e.g., double-sided tape) that is disposed in the recess 501 (see Figure 31), or at least one resilient hook 503 (see Figure 32) that is for engaging with the sensor assembly 70.

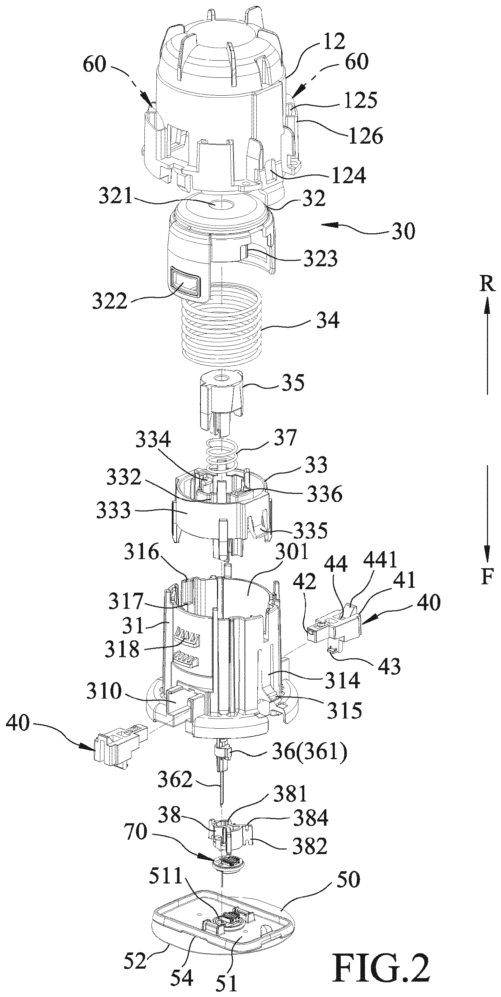

In this modification, the insertion device further includes a pair of fixing members 40. The fixing members 40 are mounted to a slide groove 310 (see Figure 2) of the main body 31 of the main body assembly 300. Each of the fixing members 40 has a push portion 41 that corresponds in position to a respective one of two stop portions 226 (see Figure 3) of the lower casing 20, a support portion 42 that is opposite to the push portion 41, a fixing hook structure 43 that is located between the push portion 41 and the support portion 42, and a cam portion 44 that is located between the push portion 41 and the support portion 42 and that has an inclined cam surface 441.

The push portion 41 of each of the fixing members 40 is limited by the respective one of the stop portions 226 of the lower casing 20, so that the fixing members 40 are positioned relative to the main body 31. The sensing seat 71 of the sensor assembly 70 is supported by the support portions 42 of the fixing members 40 so as to be positioned relative to the main body 31. The fixing hook structures 43 of the fixing members 40 respectively engage with the base hook structures 54 of the base 50.

With reference to Figures 3 and 4, before the lower casing 20 is separated from the upper casing 10, the fixing members 40 interfere with the lower casing 20 and the cover body 12 of the upper casing 10, so that the upper casing 10 is prevented from moving downwardly by the fixing members 40 to further prevent unintentional insertion operation of the insertion device.

Since the sensor assembly 70 has a lower end supported by the fixing members 40, and since the fixing members 40 engage with the base 50, the base 50 is limited within the insertion device. When the lower casing 20 is separated from the upper casing 10 (or uncovers the base 50 while being connected to the upper casing 10, in a modification), the upper casing 10 is permitted to be depressed, such that the cover body 12 moves downwardly to push and move the fixing member 40 laterally and outwardly so as to collapse the limiting structure among the fixing members 40, the sensor assembly 70 and the base 50. As such, the first elastic member 34 of the insertion module 30 is permitted to release the restoring force thereof to insert the insertion needle 36 and the sensor 72 of the sensor assembly 70 underneath the skin surface of the host, and the second elastic member 37 of the insertion module 30 is permitted to release the restoring force thereof to retract the insertion needle 36 to finish automatic-insertion and automatic-retraction operations.

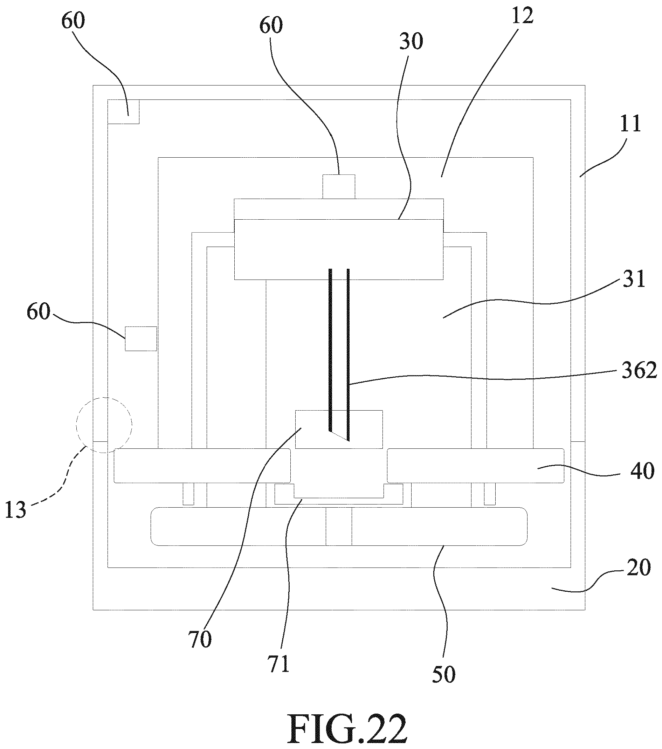

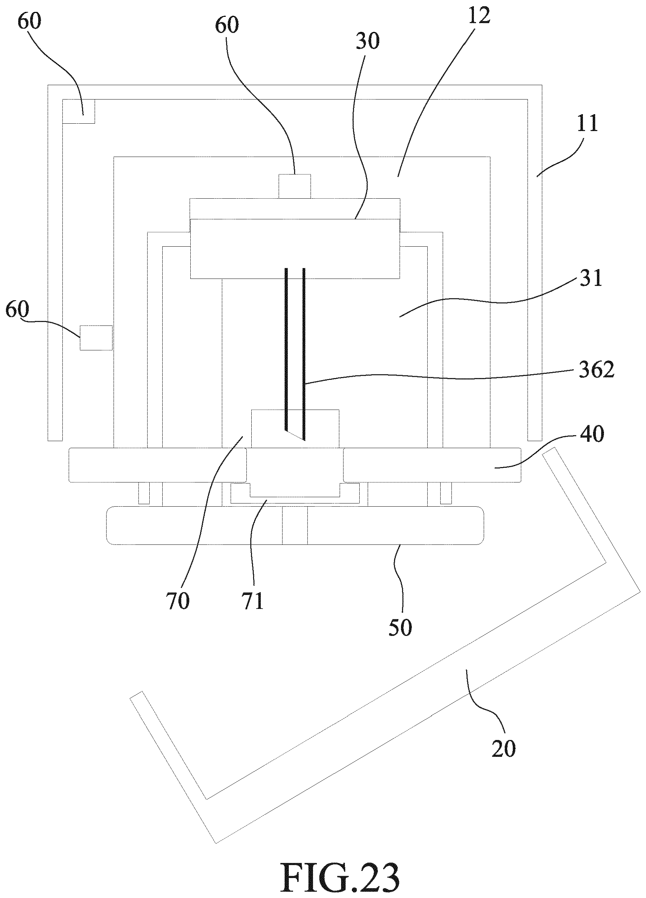

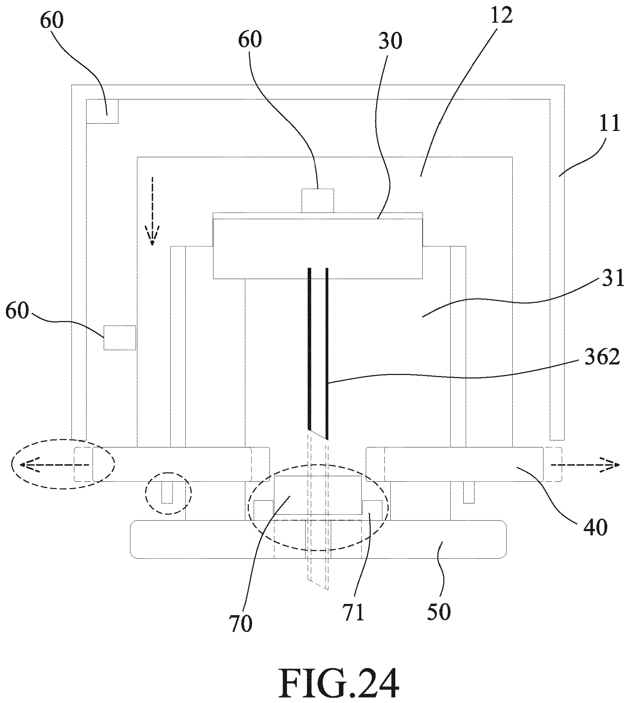

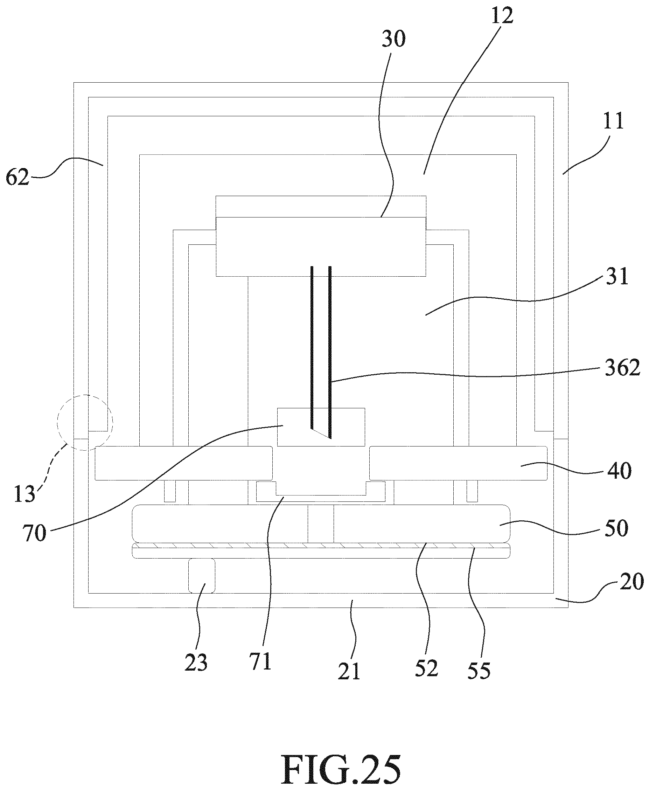

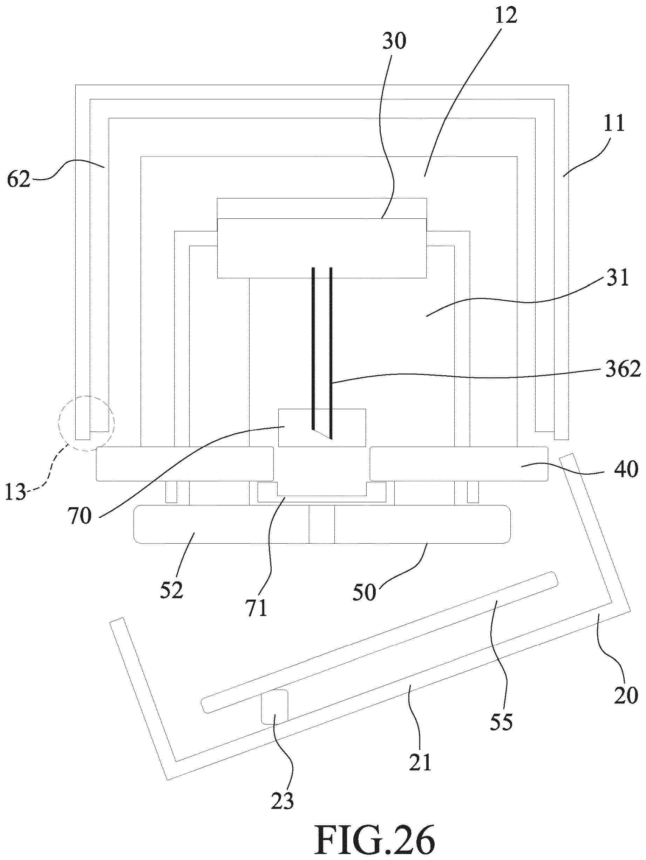

Referring to Figures 3, 4, and 22 to 26 (Figures 22 to 26 are schematic views), a second embodiment, or a modification of the first embodiment of the insertion device according to the disclosure, is for inserting a sensor into a host. The outer shell 11 and the lower casing 20 (or the upper casing 20 and the lower casing 20) are airtightly coupled to each other, so that an inner space of the insertion device is airtight. The desiccant 60 may be disposed at a suitable position anywhere in the inner space of the insertion device. In another modification, any one of the components of the insertion module 30 may be made of materials including desiccant through an injection molding technology (as shown in Figs. 22 and 23). In still another modification, an inner surface of the outer shell 11 may be provided with a desiccant layer 62 (see Figures 25 and 26), or the sensor assembly 70 may have a desiccant 60 (not shown) so that the inner space of the insertion device is kept dry and that the sensor assembly 70 is prevented from being moistened.

Referring further to Figures 25 and 26, the base 50 further includes a tearing element 23 that is fixedly mounted to a base plate 21 of the lower casing 20. The tearing element 23 is connected to the release layer 55 (see Figures 31 and 33) that is separably adhered to the adhesive pad 52. When the lower casing 20 is separated from the upper casing 10 (or uncovers the base 50 while being connected to the upper casing 10), the tearing element 23 simultaneously separates the release layer 55 from the adhesive pad 52. Since the release layer 55 is not separated from the adhesive pad 52 until the insertion device is to perform the insertion operation, the adhesive pad 52 is prevented from being stained before the insertion operation. In this embodiment, the lower casing 20 is in the form of a cap. In other embodiments, the lower casing 20 may be in the form of an aluminum foil (not shown).

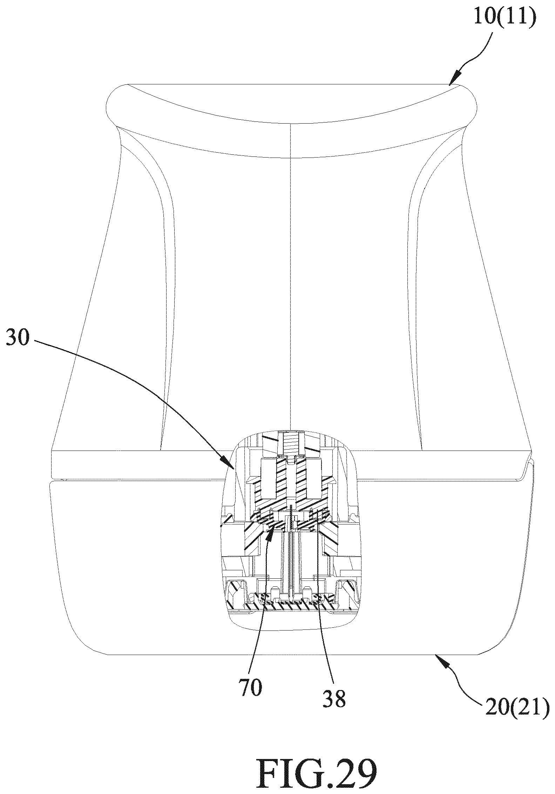

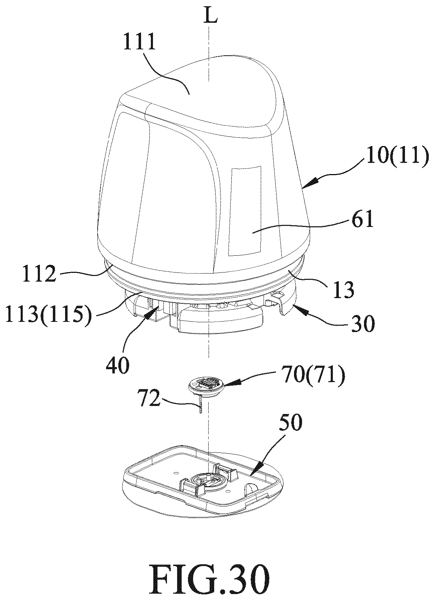

Referring to Figures 3 and 4, the outer shell 11 is sleeved onto the insertion module 30 after the components of the insertion module 30 are assembled. At this time, the sensor assembly 70 has been mounted to the insertion module 30 via the auxiliary insertion seat 38 (see Figure 29), the lower casing 20 is subsequently coupled to the outer shell 11. The sensor assembly 70 does not need to be manually adjusted during the insertion operation of the insertion device. It means that a user does not require additional manual operation of grasping the sensor assembly 70 onto the base 50 by the implanting module 30 (i.e., the insertion module 30). In this embodiment, all the components of the insertion device have been assembled in the factory, but can be disassembled and reassembled by medical personnel or a user (see Figure 30). When the insertion device is used to perform the insertion operation, the user can easily manipulate the insertion device (e.g., by simply depressing the upper casing 10) to mount the base 50 onto the skin surface of the host by the adhesive pad 52 of the base 50 followed by coupling the sensor assembly 70 onto the base 50.

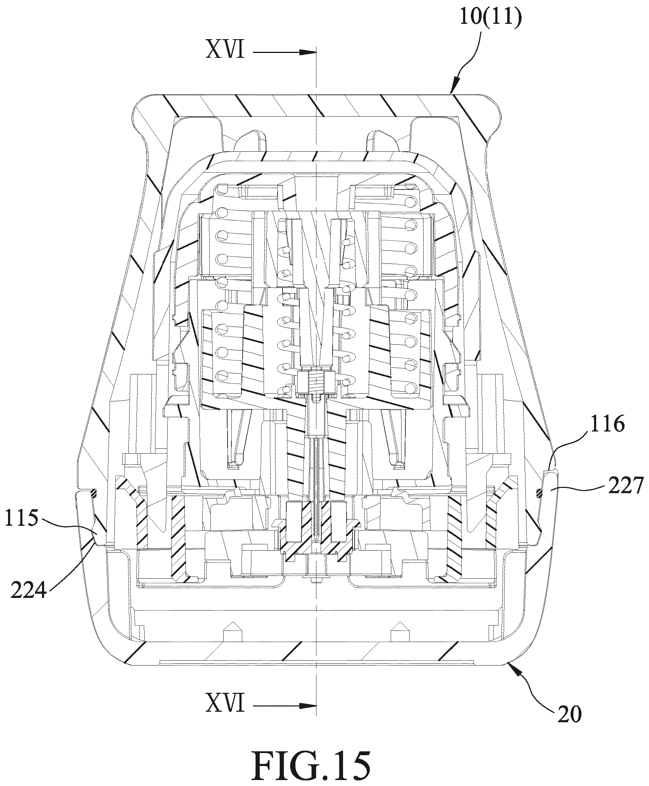

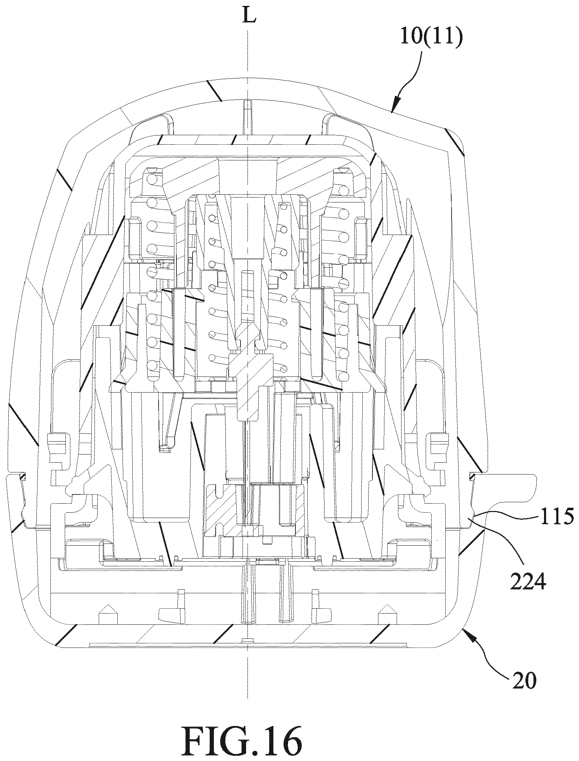

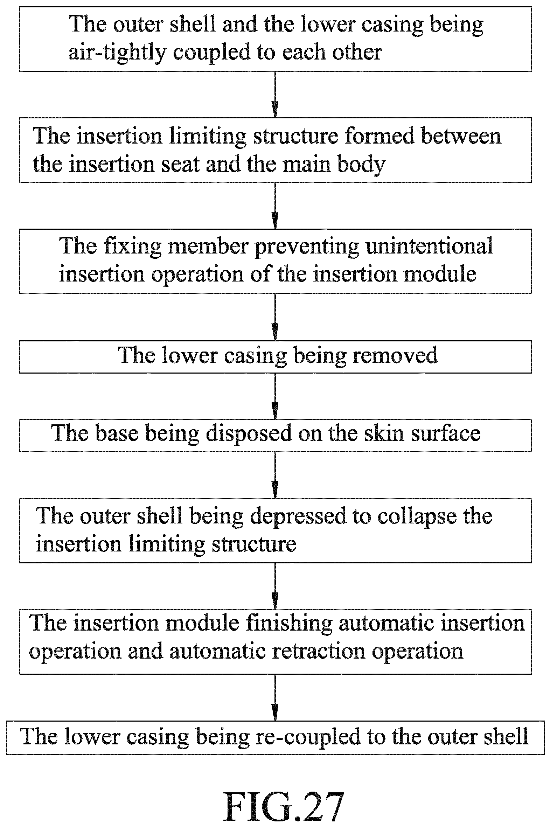

Referring to Figures 15 and 16, the separated lower casing 20 can be re-coupled to the upper casing 10. The lower casing 20 can seal a bottom portion of the outer shell 11 by the engagement between the annular protruding portion 115 of the upper casing 10 and the annular groove 224 of the lower casing 20. When the lower casing 20 is re-coupled to the outer shell 11 of the upper casing 10, the engagement between the lower positioning portion 227 of the lower casing 20 and the upper positioning portion 116 of the upper casing 10 ensures that the lower casing 20 and the upper casing 10 are properly positioned to each other. In addition, the inner space of the insertion device can serve as a waste storing space. A used base 50 can be removed from the host and be placed in the inner space of the insertion device, such that the used insertion device can be disposed in compliance with standard of discard of medical waste. The whole insertion operation of the insertion device according to the disclosure is shown in Figure 27.

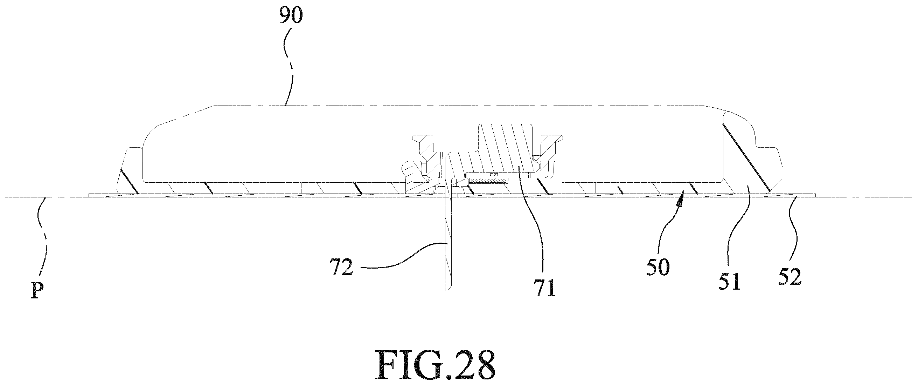

Figs. 28 shows that after the sensor 72 is implanted under the skin surface P of the living body, the sensor assembly 70 and the base 50 that are simultaneously disposed on the skin surface P of the living body, a transmitter 90 is installed on the base 50 in the form of an upper cover to process the physiological signals measured by the sensor 72 and allows the signals to be transmitted to the outside. In order to reduce the number of components to be implanted during the implantation process and also reduce the loading of the container, the inserter device of the present invention does not include a transmitter. The separately arranged the transmitter 90 and the sensor assembly 70 are to ensure the electronic parts are not damaged by the sterilization process, so the production yield of the device can be improved.

Referring to Figures 31 and 32, a third embodiment of the insertion device (similar to the automatic needle insertion and automatic needle extraction of the first embodiment, but in this embodiment does not include the outer shell 11) can optionally have a bottom cover 20, or uses a sealed package to seal the insertion device. The insertion device includes a cover body 12 and an insertion module 30.

The cover body 12 has an accommodating space 14, and a pair of casing engaging structures 124.

The insertion module 30 is disposed in the accommodating space 14, and includes a main body 31, an insertion seat 33, a first elastic member 34, a retraction seat 35, a second elastic member 37, a base 50 and a sensor assembly 70.

The main body 31 is associated with the cover body 12, and cooperates with the cover body 12 to define a displacement space 301 therebetween. The main body 31 has a pair of body engaging structures 315 that are able to respectively engage the casing engaging structure 124 of the cover body 12.

The insertion seat 33 is removably positioned in the cover body 12, and is able to move in the displacement space 301 between the main body 31 and the cover body 12 along an axial line (L).

The cover body 12 has a pair of urging portions 123 on an inner surrounding surface thereof. The main body 31 has a pair of stopping portions 317. The insertion seat 33 has a pair of buckle portions 335 that respectively and separably abut against the stopping portions 317 of the main body 31 so as to position the insertion seat 33 relative to the main body 31, and that are able to be respectively pushed by the urging portions 123 of the cover body 12 to be respectively separated from the stopping portions 317 of the main body 31. The buckle portions 335 of the insertion seat 33 respectively and separably abut the stopping portions 317 of the main body 31 so as to form an insertion limiting structure (A2) between the insertion seat 33 and the main body 31.

The insertion seat 33 further has at least one limiting groove 330. The cover body 12 further has at least one limiting member 100 that removably engages with the limiting groove 330 of the insertion seat 33. The insertion seat 33 further has at least one retraction positioning portion 334 that separably abuts against the limiting member 100 of the cover body 12.

The first elastic member 34 has two opposite ends respectively abutting against the insertion seat 33 and the cover body 12. In this embodiment, the first elastic member 34 may be configured as a pre-compressed spring.

The retraction seat 35 is mounted with an insertion needle 36, and is removably positioned relative to the insertion seat 33. The retraction positioning portion 334 of the insertion seat 33 is removably limited by the limiting member 100 of the cover body 12, so as to form a retraction limiting structure (B2) that positions the retraction seat 35 relative to the insertion seat 33.

The second elastic member 37 has two opposite ends respectively abutting against the insertion seat 33 and the retraction seat 35. In this embodiment, the second elastic member 37 may be configured as a pre-compressed spring.

The base 50 is separably positioned relative to the main body 31, and includes a base seat 51, and an adhesive pad 52 that is fixedly connected to the base seat 51.

The sensor assembly 70 is to be separably mounted to the base 50, and includes a sensing seat 71, and a sensor 72 that is mounted to the sensing seat 71 and that is separably coupled to the insertion needle 36. The base 50 further has a mounting portion 500 for the sensor assembly 70 to be separably mounted thereto, and the mounting methods are similar as those described in the previous embodiment. The insertion module 30 further includes an auxiliary insertion seat 38 that is separably mounted to the insertion needle 36. The sensor assembly 70 is separably mounted to the auxiliary insertion seat 38. The auxiliary insertion seat 38 slidably engages with the main body assembly 300 to guide the insertion needle 36 to steadily insert the sensor 72 into the host without runout or inclination so as to alleviate pain of the host.

Referring to Figure 33, a fourth embodiment of the insertion device according to the disclosure (similar to the third embodiment but the insertion module includes independent main cover) includes a cover body 12 and an insertion module 30.

The cover body 12 has an accommodating space 14, and a pair of casing engaging structures 124.

The insertion module 30 is disposed in the accommodating space 14, and includes a main body 31, a main cover 32, an insertion seat 33, a first elastic member 34, a retraction seat 35, a second elastic member 37, a base 50 and a sensor assembly 70.

The main body 31 is connected to the main cover 32, and cooperates with the main cover 32 to define a displacement space 301 therebetween. The main body 31 has a pair of body engaging structures 315 that are able to respectively engage the casing engaging structure 124 of the cover body 12. A top portion of the cover body 12 and a top portion of the main cover 32 is spaced apart from each other by a distance (D).

The insertion seat 33 is removably positioned in the main cover 32, and is able to move in the displacement space 301 between the main body 31 and the main cover 32 along an axial line (L).

The cover body 12 has a pair of urging portions 123 on an inner surrounding surface thereof. The main body 31 has a pair of stopping portions 317. The insertion seat 33 has a pair of buckle portions 335 that respectively and separably abut against the stopping portions 317 of the main body 31 so as to position the insertion seat 33 relative to the main body 31, and that are able to be respectively pushed by the urging portions 123 of the cover body 12 to be respectively separated from the stopping portions 317 of the main body 31. The buckle portions 335 of the insertion seat 33 respectively and separably abut the stopping portions 317 of the main body 31 so as to form an insertion limiting structure (A1) between the insertion seat 33 and the main body 31.

The insertion seat 33 further has at least one limiting groove 330. The main cover 32 further has at least one limiting member 320 that removably engages with the limiting groove 330 of the insertion seat 33. The insertion seat 33 further has at least one retraction positioning portion 334 that separably abuts against the limiting member 320 of the main cover 32.

The first elastic member 34 has two opposite ends respectively abutting against the insertion seat 33 and the main cover 32. In this embodiment, the first elastic member 34 may be configured as a pre-compressed spring.

The retraction seat 35 is mounted with an insertion needle 36, and is removably positioned relative to the insertion seat 33. The retraction positioning portion 334 of the insertion seat 33 is removably limited by the limiting member 320 of the main cover 32, so as to form a retraction limiting structure (B1) that positions the retraction seat 35 relative to the insertion seat 33.

The second elastic member 37 has two opposite ends respectively abutting against the insertion seat 33 and the retraction seat 35. In this embodiment, the second elastic member 37 may be configured as a pre-compressed spring.

The base 50 is separably positioned relative to the main body 31, and includes a base seat 51, and an adhesive pad 52 that is fixedly connected to the base seat 51.

The sensor assembly 70 is to be separably mounted to the base 50, and includes a sensing seat 71, and a sensor 72 that is mounted to the sensing seat 71 and that is separably coupled to the insertion needle 36. The base 50 further has a mounting portion 500 for the sensor assembly 70 to be separably mounted thereto, and the mounting methods are similar as those described in the previous embodiment.

The insertion module 30 further includes an auxiliary insertion seat 38 that is separably mounted to the insertion needle 36. The sensor assembly 70 is separably mounted to the auxiliary insertion seat 38. The auxiliary insertion seat 38 slidably engages with the main body assembly 300 to guide the insertion needle 36 to steadily insert the sensor 72 into the host without runout or inclination so as to alleviate pain of the host.

The insertion device according to the disclosure (the first to fourth embodiments and the modifications thereof) is operated in such a manner that the upper casing 10 or the cover body 12 is depressed so as to collapse the insertion limiting structure (A, A1, A2), and the first elastic member 34 therefore releases the restoring force to move the insertion seat 33 downwardly to perform the automatic-insertion operation. In other words, in this disclosure, the insertion needle 36 is driven to be inserted into the host by the first elastic member 34 rather than by manual depression of a user. Therefore, the insertion operation and the retraction operation can be smoothly performed by any user even if the user is not an adept operator.

The insertion operation performed by the third and fourth embodiments, according to the disclosure are operated via a single-action operation. The insertion operation of the sensor 72 into the host is illustrated as follows:
Referring to Figures 31 to 33, when the adhesive pad 52 is adhered to a skin surface of the host and the cover body 12 is not depressed, the insertion limiting structure (A1, A2) that is formed between the buckle portions 335 of the insertion seat 33 and the stopping portions 317 of the main body 31 maintains the insertion seat 33 at a pre-insertion position. When the cover body 12 is depressed toward the skin surface, the buckle portions 335 of the insertion seat 33 are respectively pushed by the urging portions 123 of the cover body 12 to be deformed inwardly and to be respectively separated from the stopping portions 317 of the main body 31, so that the insertion limiting structure (A1, A2) is collapsed. At the same time, the body engaging structures 315 of the main body 31 respectively engage the casing engaging structure 124 of the cover body 12, so that the cover body 12 is positioned relative to the main body 31.

After the insertion limiting structure (A1, A2) is collapsed, the restoring force of the first elastic member 34 is permitted to be released, and moves the insertion seat 33 to an insertion position to implement automatic-insertion, such that the sensor assembly 70 is moved by the insertion seat 33 to a post-insertion position, that the sensor 72 is inserted underneath the skin surface, and that the sensing seat 71 is positioned onto the mounting portion 500 of the base 50. After the sensor 72 is inserted underneath the skin surface, the limiting member 320 of the main cover 32 or the limiting member 100 of the cover body 12 is separated from the limiting groove 330 and the retraction positioning portion 334 of the insertion seat 33, so that the retraction positioning portion 334 is permitted to be deformed outwardly to collapse the retraction limiting structure (B1, B2). As such, the restoring force of the second elastic member 37 is permitted to be released, and drives the retraction seat 35 to move past the retraction positioning portion 334 of the insertion seat 33 away from the skin surface, such that the insertion needle 36 is separated from the auxiliary insertion seat 38 and is retracted into the insertion seat 33 to hide a needle 362 thereof and to implement automatic-retraction. At this time, the coupling portions 383 of the auxiliary insertion seat 38 is maintained to be fitted into the coupling portion 711 of the sensing seat 71 so that the auxiliary insertion seat 38 is connected to the sensing seat 71 of the sensor assembly 70.

Afterward, the cover body 12 and the insertion module 30 are separated from the base 50 and the skin surface of the host, so as to finish the insertion operation of the third and fourth embodiments of the insertion device including the automatic-insertion and automatic-retraction operations.

Accordingly, the third and fourth embodiments of the insertion device may further include a lower casing (not shown) that is air-tightly and separably coupled to the cover body 12 so as to form an airtight space within the insertion device. A desiccant may be provided in the inner space of the insertion device so as to prevent the sensor 72 of the sensor assembly 70 from being moistened.

In the third or fourth embodiments, a time needed for performing the automatic-insertion operation and the automatic-retraction operation (i.e., the duration for which the insertion needle 36 is inserted into the host) is no more than 100 milliseconds, no more than 50 milliseconds, no more than 8 milliseconds, no more than 6 milliseconds, no more than 4 milliseconds or no more than 2 milliseconds;

In the fourth embodiment, by virtue of the main cover 32 that cooperates with the insertion seat 33 to compress the first elastic member 34 therebetween, the restoring force of the first elastic member 34 would not act on the cover body 12 when a user depresses the cover body 12, rendering the operation of the insertion device smooth. The insertion module further includes an insertion seat 33 and a retraction seat 35. The insertion limiting structure (A1, A2) is formed between the insertion seat 33 and the main body 31. The retraction limiting structure (B1, B2) is formed among the insertion seat 33, the retraction seat 35 and the main cover 31 or among the insertion seat 33, the retraction seat 35 and the cover body 12.

The retraction seat 35 includes an insertion needle 36. The insertion seat 33 includes a pushing portion (with reference to the reference numeral 337 in Figure 3). After the automatic-insertion operation performed by the insertion module 30, the insertion needle 36 is inserted through the base 50 to insert a sensor 72 underneath a skin surface of a host, and the pushing portion of the insertion seat 33 tightly abuts against the sensor assembly 70 (via an auxiliary insertion seat 38) so as to prevent the sensor assembly 70 from being separated from a mounting portion 500 of the base 50.

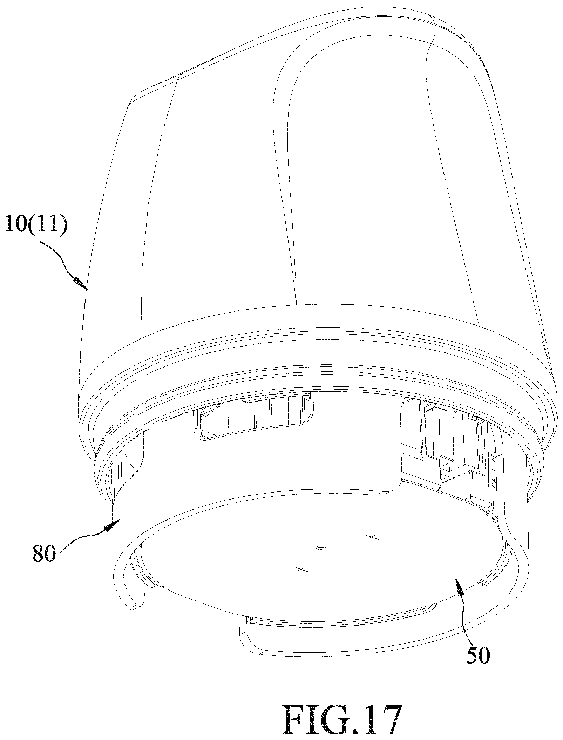

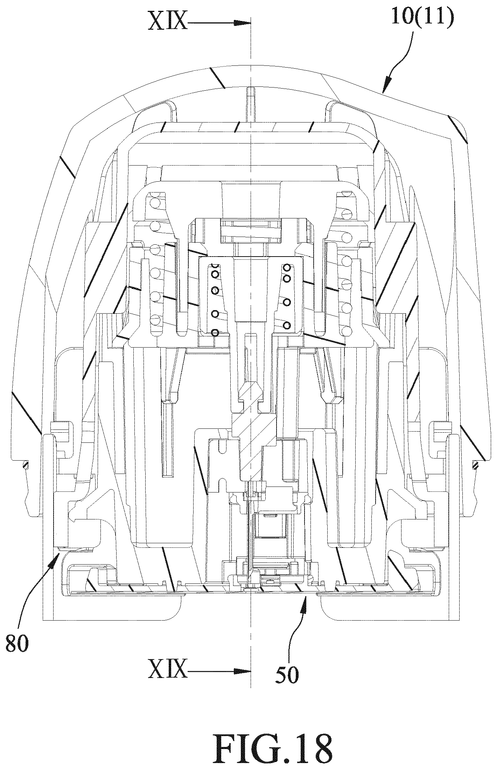

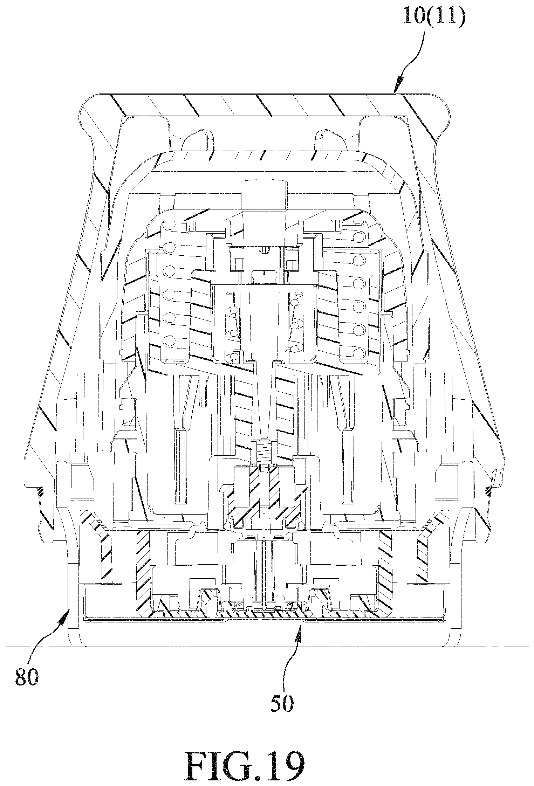

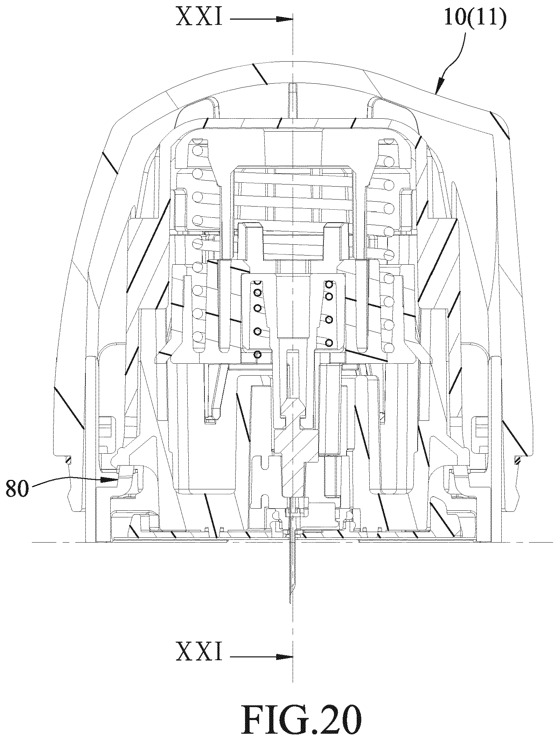

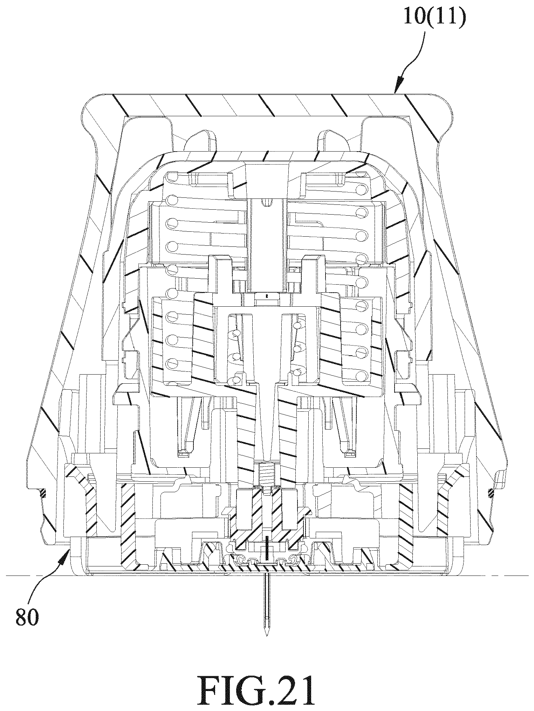

The advantages of the insertion device according to the disclosure are as follows:
1. The insertion device employs the pre-compressed first and second elastic members 34, 37 to perform the automatic-insertion and automatic-retraction operations, so that insertion device is operated via a single-action operation (i.e., by depressing the upper casing 10, outer shell 11 or the cover body 12) to finish the automatic-insertion and automatic-retraction operations. The insertion needle 36 is not driven to be inserted into the host by manual depression of a user. Therefore, the insertion operation and the retraction operation can be smoothly performed by any user even if the user is not an adept operator.
2. The lower casing 20 cooperates with the upper casing 10 or the cover body 12 to form the airtight space that is provided with the desiccant 60 therein, so that the sensor 72 of the sensor assembly 70 is prevented from being moistened to ensure the accuracy of the sensor assembly 70. In addition, a desiccation indicator 61 (see Figure 4) may be provided on inner or outer side of the outer shell 11 of the upper casing 10. Preferably, the desiccation indicator 61 may be an aridity indication material. The outer shell 11 may have a transparent or translucent portion through which the desiccation indicator 61 is visible, so a user can perceive whether or not the sensor assembly 70 is moistened. The manner in which the desiccant 60 is disposed is not limited to the disclosed embodiments, and the upper casing 10 and the lower casing 20 are not limited to be interconnected in a hard-interference manner.
3. Before the lower casing 20 is separated(or opened) from the upper casing 10 or from the cover body 12, the abutment portion 228 of the lower casing 20 limits movement of the casing engaging structure 124 of the cover body 12 or the upper casing 10, such that the upper casing 10 or the cover body 12 cannot move downwardly so as to prevent unintentional insertion operation of the insertion device due to unintentionally applying force to the insertion device.
4. After the automatic-retraction operation, the insertion needle 36 is separated from the auxiliary insertion seat 38 and is retracted into the insertion seat 33 to hide a needle 362.
5. By virtue of the main cover 32 that cooperates with the insertion seat 33 to compress the first elastic member 34 therebetween, the restoring force of the first elastic member 34 would not act on the upper casing 10 or the cover body 12 when a user depresses the upper casing 10 or the cover body 12, rendering the operation of the insertion device smooth.
6. The auxiliary insertion seat 38 slidably engages with the main body assembly 300 to guide the insert direction of the insertion needle 36 to steadily insert the sensor72 into the host without runout or inclination so as to alleviate pain of the host.
7. The base 50 has a mounting portion 500 for the sensor assembly 70 to be separably mounted thereto. The mounting portion 500 may include a recess 501 that permits the sensor assembly 70 to be press-fitted thereinto, and/or an adhesive layer 502 (e.g., double-sided tape) that is disposed in the recess 501 for the sensor assembly 70 to be adhered thereto, or at least one resilient hook 503 that is for engaging with the sensor assembly 70.
8. After the upper casing 10 (or housing 11) or the cover body 12 is depressed, a time needed for performing the automatic-insertion operation and the automatic-retraction operation is no more than 100 milliseconds, no more than 50 milliseconds, no more than 8 milliseconds, no more than 6 milliseconds, no more than 4 milliseconds or no more than 2 milliseconds.
9. In assembling the insertion device, the first elastic member 34, the retraction seat 35 , the second elastic member 37 and the insertion seat 33 are firstly disposed between the main cover 32 and the main body 31, and the auxiliary insertion seat 38 and the sensor assembly 70 are mounted to the insertion needle 36 before the insertion needle 36 is mounted to the retraction seat 35. Whereby the sensor assembly 70 and the insertion module 30 forming a clutch design, which can not only greatly improve the assembly yield, but also effectively reduce the cost of the sensor assembly.
10. The sensor assembly 70 has been mounted to the insertion module 30 via the auxiliary insertion seat 38 (see Figure 29), so the sensor assembly 70 does not require additional manual operation of grasping the sensor assembly 70 onto the base50 by the implanting module 30.
11. An inner side of a bottom portion of the upper casing 10 may be provided with a cushion wall 80 (see Figures 17 to 21) that is substantially ring-shaped. Before the insertion device is operated, the base 50 is higher than a bottom edge of the cushion wall 80. When the bottom edge of the cushion wall 80 abuts against the skin surface of the host, the base 50 is spaced apart from the skin surface, so a user can move the insertion device along the skin surface to a desired position, an depress the upper casing 10. The cushion wall 80 retracts into the upper casing 10 upon the depression, such that the base 50 can be mounted onto the skin surface. By virtue of the cushion wall 80, the insertion device can be moved along the skin surface to a desired position with the cushion wall 80 abutting against the skin surface, rendering the insertion operation stable.
12. When the lower casing 20 is re-coupled to the outer shell 11 of the upper casing 10, the engagement between the lower positioning portion 227 of the lower casing 20 and the upper positioning portion 116 of the upper casing 10 ensures that the lower casing 20 and the upper casing 10 are properly positioned to each other.

In addition to the embodiments described above, this disclosure further discloses a plurality of embodiments as defined by the claims, with each embodiment comprising the claim elements of the respective claim and the claim elements of any claim upon which the respective claim depends.

In the description above, for the purposes of explanation, numerous specific details have been set forth in order to provide a thorough understanding of the embodiments. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, in the practice of the disclosure.

## Claims

1. An insertion device adapted for a biosensor, comprising:
an upper casing (10) having a casing engaging structure (124) at a lower portion thereof;
a lower casing (20) separably connected to said upper casing (10), said lower casing (20) having an abutment portion (228) at an inner surrounding surface thereof that abuts against an outer side of said casing engaging structure (124) of said upper casing (10); and
an insertion module (30) disposed in said upper casing (10), forming movement relationship with said upper casing (10), and including a main body assembly (300), said main body assembly (300) having a body engaging structure (315) that abuts against an inner side of said casing engaging structure (124) of said upper casing (10) and that is able to engage said casing engaging structures (124) when said upper casing (10) is depressed;
wherein, when said lower casing (20) is coupled to said upper casing (10), said abutment portion (228) of said lower casing (20) limits movement of said casing engaging structure (124) of said upper casing (10), such that said upper casing (10) cannot move downwardly so as to prevent unintentional insertion operation of said insertion device;
said insertion device being **characterized in that**:
said main body assembly (300) of said insertion module (30) includes a main body (31), and a main cover (32) that is connected to said main body (31) and that cooperates with said main body (31) to define a displacement space (301) therebetween, said upper casing (10) including an outer shell (11), and a cover body (12) that is disposed in said outer shell (11), said casing engaging structure (124) being disposed at a lower portion of said cover body (12);
said insertion module (30) includes an insertion seat (33) that is disposed in said displacement space (301), a first elastic member (34) that has two opposite ends respectively abutting against said insertion seat (33) and said main cover (32), a retraction seat (35) that is positioned relative to said insertion seat (33), and a second elastic member (37) that has two opposite ends respectively abutting against said insertion seat (33) and said retraction seat (35);
said cover body (12) of said upper casing (10) has at least one urging portion (123) at an inner surrounding surface thereof, said insertion seat (33) having at least one buckle portion (335) that separably abuts against a stopping portion (317) of said main body (31) so as to form an insertion limiting structure (A) between said insertion seat (33) and said main body (31);
wherein, when said lower casing (20) is separated from said upper casing (10) and when said upper casing (10) is depressed toward a skin surface, said at least one buckle portion (335) of said insertion seat (33) is pushed by said at least one urging portion (123) of said upper casing (10) to be deformed inwardly and to be separated from said stopping portion (317) of said main body (31) of said main body assembly (300), so that said insertion limiting structure (A) is collapsed; and
wherein, after depression of said cover body (12), said casing engaging structure (124) of said upper casing (10) engages said body engaging structure (315) of said main body assembly (300) such that said upper casing (10) is positioned relative to said main body assembly (300), and said at least one urging portion (123) of said upper casing (10) is stuck underneath said main body (31) such that said cover body (12) cannot be moved upward.

2. The insertion device as claimed in claim 1, wherein said body engaging structure (315) is disposed on said main body (31) of said main body assembly (300), said body engaging structure (315) being able to engage with said casing engaging structure (124).

3. The insertion device as claimed in claim 1, further comprising a fixing member (40), said fixing member (40) being slidably mounted to said main body (31) of said main body assembly (300), said fixing member (40) interfering with said cover body (12) and said lower casing (20) so as to prevent unintentional insertion operation of said insertion device when said lower casing (20) is coupled to said upper casing (10).

4. The insertion device as claimed in claim 3, wherein said fixing member (40) has a push portion (41) that corresponds in position to said lower casing (20), a support portion (42) that is opposite to said push portion (41), a fixing hook structure (43) that is located between said push portion (41) and said support portion (42), and a cam portion (44) that is located between said push portion (41) and said support portion (42).

5. The insertion device as claimed in claim 4, further comprising a sensor assembly (70) that is coupled to said insertion module (30) and that is supported by said support portion (42) of said fixing member (40).

6. The insertion device as claimed in claim 5, further comprising a base (50), said base (50) having a base hook structures (54) that corresponds to said fixing hook structure (43) of said fixing member (40), said base hook structures (54) engaging with said fixing hook structure (43) of said fixing member (40) to position said base (50) in said insertion device, said sensor assembly (70) being separated from said insertion module (30) and positioned onto said base (50) during the insertion operation of said insertion device.

7. The insertion device as claimed in claim 5, further comprising an insertion needle (36) that is coupled to and moved by said insertion module (30), said insertion needle (36) being coupled to an auxiliary insertion seat (38), said sensor assembly (70) being separably mounted to said auxiliary insertion seat (38).

8. The insertion device as claimed in claim 1, wherein said insertion seat (33) has a limiting groove (330), said main cover (32) having a limiting member (323) that removably engages with said limiting groove (330) of said insertion seat (33), said insertion seat (33) further having a retraction positioning portion (334) that limits movements of said retraction seat (35) relative to said insertion seat (33), said retraction positioning portion (334) of said insertion seat (33) being limited by said limiting member (323) of said main cover (32), so as to form a retraction limiting structure (B) among said retraction seat (35), said insertion seat (33) and said main cover (32) of said main body assembly (300).

9. The insertion device as claimed in claim 8, wherein when said insertion seat (33) of said insertion module (30) moves downwardly to perform an automatic-insertion operation, said limiting member (323) of said main cover (32) is separated from said limiting groove (330), so that a structural limitation between said insertion seat (33) and said main cover (32) is collapsed.

10. The insertion device as claimed in claim 9, wherein when the structural limitation between said insertion seat (33) and said main cover (32) is collapsed, said retraction positioning portion (334) is permitted to be deformed outwardly to collapse the retraction limiting structure (B), so as to collapse a structural limitation between said retraction seat (35) and said insertion seat (33).

11. The insertion device as claimed in claim 6, wherein said base (50) has a mounting portion (500) for said sensing assembly (70) to be separably mounted thereto, said mounting portion (500) of said base (50) including a recess (501) that permits said sensing assembly (70) to be press-fitted thereinto, an adhesive layer (502) that is disposed in said recess (501) for said sensing assembly (70) to be adhered thereto, and at least one resilient hook (503) that is for engaging with said sensing assembly (70).

12. The insertion device as claimed in claim 1, wherein said lower casing (20) is air-tightly and separably coupled to said upper casing (10) so as to formed an airtight space within said insertion device, said airtight space being provided with a desiccant therein.

13. The insertion device as claimed in claim 6, further comprising a cushion wall (80) that is disposed on an inner side of a bottom portion of said upper casing (10), said base (50) being higher than a bottom edge of said cushion wall (80) before said insertion device is operated.

## Patentansprüche

1. Einführvorrichtung, die für einen Biosensor geeignet ist, umfassend:
ein oberes Gehäuse (10), das an seinem unteren Abschnitt eine Gehäuseeingriffsstruktur (124) aufweist;
ein unteres Gehäuse (20), das lösbar mit dem oberen Gehäuse (10) verbunden ist, wobei das untere Gehäuse (20) an einer seiner inneren Umfangsoberflächen einen Anschlagabschnitt (228) aufweist, der an einer Außenseite der Gehäuseeingriffsstruktur (124) des oberen Gehäuses (10) anliegt; und
ein in dem oberen Gehäuse (10) angeordnetes Einführmodul (30), das eine Bewegungsbeziehung mit dem oberen Gehäuse (10) bildet, und das eine Hauptkörperbaugruppe (300) einschließt, wobei die Hauptkörperbaugruppe (300) eine Körpereingriffsstruktur (315) aufweist, die an einer Innenseite der Gehäuseeingriffsstruktur (124) des oberen Gehäuses (10) anliegt und in der Lage ist, mit den Gehäuseeingriffsstrukturen (124) in Eingriff zu kommen, wenn das obere Gehäuse (10) nach unten gedrückt wird;
wobei, wenn das untere Gehäuse (20) mit dem oberen Gehäuse (10) verbunden ist, der Anschlagabschnitt (228) des unteren Gehäuses (20) die Bewegung der Gehäuseeingriffsstruktur (124) des oberen Gehäuses (10) begrenzt, sodass sich das obere Gehäuse (10) nicht nach unten bewegen kann, um einen unbeabsichtigten Einführvorgang der Einführvorrichtung zu verhindern;
wobei die Einführvorrichtung **dadurch gekennzeichnet ist, dass**:
die Hauptkörperbaugruppe (300) des Einführmoduls (30) einen Hauptkörper (31) einschließt, sowie eine Hauptabdeckung (32), die mit dem Hauptkörper (31) verbunden ist und mit diesem (31) zusammenwirkt, um dazwischen einen Verschiebungsraum (301) zu bilden, wobei das obere Gehäuse (10) eine Außenhülle (11) sowie einen Abdeckungskörper (12) einschließt, der in der Außenhülle (11) angeordnet ist, wobei die Gehäuseeingriffsstruktur (124) an einem unteren Abschnitt des Abdeckungskörpers (12) angeordnet ist;
das Einführmodul (30) einen Einführsitz (33) einschließt, der in dem Verschiebungsraum (301) angeordnet ist, ein erstes elastisches Element (34), das zwei gegenüberliegende Enden aufweist, die jeweils an dem Einführsitz (33) und der Hauptabdeckung (32) anliegen, einen Rückzugssitz (35), der relativ zu dem Einführsitz (33) positioniert ist, sowie ein zweites elastisches Element (37), dessen zwei gegenüberliegende Enden jeweils an dem Einstecksitz (33) und dem Rückzugssitz (35) anliegen;
der Abdeckungskörper (12) des oberen Gehäuses (10) an seiner inneren Umfangsoberfläche mindestens einen Andrückabschnitt (123) aufweist, wobei der Einführsitz (33) mindestens einen Rastabschnitt (335) aufweist, der lösbar an einem Anschlagabschnitt (317) des Hauptkörpers (31) anliegt, um eine Einführbegrenzungsstruktur (A) zwischen dem Einfürsitz (33) und dem Hauptkörper (31) zu bilden;
wobei, wenn das untere Gehäuse (20) vom oberen Gehäuse (10) getrennt ist und das obere Gehäuse (10) in Richtung einer Hautoberfläche gedrückt wird, der mindestens eine Rastabschnitt (335) des Einstecksitzes (33) durch den mindestens einen Druckabschnitt (123) des oberen Gehäuses (10) nach innen verformt und von dem Anschlagabschnitt (317) des Hauptkörpers (31) der Hauptkörperbaugruppe (300) getrennt wird, sodass die Einführbegrenzungsstruktur (A) zusammenfällt; und
wobei nach dem Herunterdrücken des Abdeckungskörpers (12) die Gehäuseeingriffsstruktur (124) des oberen Gehäuses (10) in die Körpereingriffsstruktur (315) der Hauptkörperbaugruppe (300) eingreift, sodass das obere Gehäuse (10) relativ zur Hauptkörperbaugruppe (300) positioniert ist, und der mindestens eine Druckabschnitt (123) des oberen Gehäuses (10) unter dem Hauptkörper (31) festgeklemmt wird, sodass der Abdeckungskörper (12) nicht nach oben bewegt werden kann.

2. Einführvorrichtung nach Anspruch 1, wobei die mit Körpereingriffsstruktur (315) am Hauptkörper (31) der Hauptkörperbaugruppe (300) angeordnet ist, wobei die Körpereingriffsstruktur (315) mit Gehäuseeingriffsstruktur (124) in Eingriff kommen kann.

3. Einführvorrichtung nach Anspruch 1, ferner umfassend ein Befestigungselement (40), wobei das Befestigungselement (40) verschiebbar an dem Hauptkörper (31) der Hauptkörperbaugruppe (300) angebracht ist, wobei das Befestigungselement (40) in den Abdeckungskörper (12) und das untere Gehäuse (20) eingreift, um einen unbeabsichtigten Einführvorgang der Einführvorrichtung zu verhindern, wenn das untere Gehäuse (20) mit dem oberen Gehäuse (10) verbunden ist.

4. Einführvorrichtung nach Anspruch 3, wobei das Befestigungselement (40) einen Druckabschnitt (41), der in seiner Position dem unteren Gehäuse (20) entspricht, einen Stützabschnitt (42), der dem Druckabschnitt (41) gegenüberliegt, eine Befestigungshakenstruktur (43), die sich zwischen dem Druckabschnitt (41) und dem Stützabschnitt (42) befindet, sowie einen Nockenabschnitt (44) aufweist, der sich zwischen dem Druckabschnitt (41) und dem Stützabschnitt (42) befindet.

5. Einführvorrichtung nach Anspruch 4, ferner umfassend eine Sensorbaugruppe (70), die mit dem Einführmodul (30) verbunden ist und von dem Stützabschnitt (42) des Befestigungselements (40) getragen wird.

6. Einführvorrichtung nach Anspruch 5, ferner umfassend eine Basis (50), wobei die Basis (50) eine Basishakenstruktur (54) aufweist, die der Befestigungshakenstruktur (43) des Befestigungselements (40) entspricht, wobei die Basishakenstruktur (54) mit der Befestigungshakenstruktur (43) des Befestigungselements (40) in Eingriff kommt, um die Basis (50) in der Einführvorrichtung zu positionieren, wobei die Sensorbaugruppe (70) während des Einführvorgangs der Einführvorrichtung vom Einführmodul (30) getrennt und auf der Basis (50) positioniert wird.

7. Einführvorrichtung nach Anspruch 5, ferner umfassend eine Einführnadel (36), die mit dem Einführmodul (30) gekoppelt ist und von diesem bewegt wird, wobei die Einführnadel (36) mit einem zusätzlichen Einführsitz (38) verbunden ist, wobei die Sensorbaugruppe (70) lösbar an dem Hilfseinführsitz (38) angebracht ist.

8. Einführvorrichtung nach Anspruch 1, wobei der Einführsitz (33) eine Begrenzungsnut (330) aufweist, wobei die Hauptabdeckung (32) ein Begrenzungselement (323) aufweist, das lösbar in die Begrenzungsnut (330) des Einführsitzes (33) in Eingriff kommt, wobei der Einführsitz (33) ferner einen Rückzugspositionierungsabschnitt (334) aufweist, der Bewegungen des Rückzugsitzes (35) relativ zum Einführsitz (33) begrenzt, wobei der Rückzugspositionierungsabschnitt (334) des Einführsitzes (33) durch das Begrenzungselement (323) der Hauptabdeckung (32) begrenzt wird, sodass zwischen dem Rückzugssitz (35), dem Einführzsitz (33) und der Hauptabdeckung (32) der Hauptkörperbaugruppe (300) eine Rückzugsbegrenzungsstruktur (B) gebildet wird.

9. Einführvorrichtung nach Anspruch 8, wobei, wenn sich der Einführsitz (33) des Einführmoduls (30) zur Durchführung eines automatischen Einführvorgangs nach unten bewegt, das Begrenzungselement (323) der Hauptabdeckung (32) von der Begrenzungsnut (330) getrennt wird, sodass eine strukturelle Begrenzung zwischen dem Einführsitz (33) und der Hauptabdeckung (32) aufgehoben wird.

10. Einführvorrichtung nach Anspruch 9, wobei, wenn die strukturelle Begrenzung zwischen dem Einführsitz (33) und der Hauptabdeckung (32) zusammenfällt, der Rückzugspositionierungsabschnitt (334) sich nach außen verformen kann, um die Rückzugsbegrenzungsstruktur (B) zusammenfallen zu lassen, um dadurch eine strukturelle Begrenzung zwischen dem Rückzugssitz (35) und dem Einführsitz (33) zusammenzufalten.

11. Einführvorrichtung nach Anspruch 6, wobei die Basis (50) einen Befestigungsabschnitt (500) aufweist, an dem die Sensorbaugruppe (70) lösbar befestigt werden kann, wobei der Befestigungsabschnitt (500) der Basis (50) eine Aussparung (501) einschließt, die ein Einpressen der Sensorbaugruppe (70) in diese ermöglicht, sowie eine Klebeschicht (502), die in der Aussparung (501) angeordnet ist, damit die Sensorbaugruppe (70) daran befestigt werden kann, und mindestens einen elastischen Haken (503), der zum Eingriff mit der Sensorbaugruppe (70) dient.

12. Einführvorrichtung nach Anspruch 1, wobei das untere Gehäuse (20) luftdicht und lösbar mit dem oberen Gehäuse (10) verbunden ist, sodass innerhalb der Einführvorrichtung ein luftdichter Raum gebildet wird, wobei dieser luftdichte Raum mit einem Trockenmittel gefüllt ist.

13. Einführvorrichtung nach Anspruch 6, ferner umfassend eine Polsterwand (80), die an einer Innenseite eines Bodenabschnitts des oberen Gehäuses (10) angeordnet ist, wobei die Basis (50) vor der Betätigung der Einführvorrichtung höher ist als eine Unterkante der Polsterwand (80).

## Revendications

1. Dispositif d'insertion conçu pour un biocapteur, comprenant :
un boîtier supérieur (10) ayant une structure de mise en prise de boîtier (124) au niveau d'une partie inférieure de celui-ci ;
un boîtier inférieur (20) relié séparément audit boîtier supérieur (10), ledit boîtier inférieur (20) ayant une partie de butée (228) au niveau d'une surface périphérique interne de celui-ci qui vient en butée contre une face externe de ladite structure de mise en prise de boîtier (124) dudit boîtier supérieur (10) ; et
un module d'insertion (30) disposé dans ledit boîtier supérieur (10), formant une relation de mouvement avec ledit boîtier supérieur (10), et comportant un ensemble corps principal (300), ledit ensemble corps principal (300) ayant une structure de mise en prise de corps (315) qui vient en butée contre une face interne de ladite structure de mise en prise de boîtier (124) dudit boîtier supérieur (10) et qui est capable de se mettre en prise avec lesdites structures de mise en prise de boîtier (124) lorsque ledit boîtier supérieur (10) est enfoncé ;
dans lequel, lorsque ledit boîtier inférieur (20) est accouplé audit boîtier supérieur (10), ladite partie de butée (228) dudit boîtier inférieur (20) limite le mouvement de ladite structure de mise en prise de boîtier (124) dudit boîtier supérieur (10), de telle sorte que ledit boîtier supérieur (10) ne puisse pas se déplacer vers le bas de manière à empêcher une opération d'insertion involontaire dudit dispositif d'insertion ;
ledit dispositif d'insertion étant **caractérisé en ce que** :
ledit ensemble corps principal (300) dudit module d'insertion (30) comporte un corps principal (31) et un couvercle principal (32) qui est relié audit corps principal (31) et qui coopère avec ledit corps principal (31) pour définir un espace de déplacement (301) entre eux, ledit boîtier supérieur (10) comportant une coque externe (11), et un corps de couvercle (12) qui est disposé dans ladite coque externe (11), ladite structure de mise en prise de boîtier (124) étant disposée au niveau d'une partie inférieure dudit corps de couvercle (12) ;
ledit module d'insertion (30) comporte un siège d'insertion (33) qui est disposé dans ledit espace de déplacement (301), un premier élément élastique (34) qui a deux extrémités opposées venant respectivement en butée contre ledit siège d'insertion (33) et ledit couvercle principal (32), un siège de rétraction (35) qui est positionné par rapport audit siège d'insertion (33), et un second élément élastique (37) qui a deux extrémités opposées venant respectivement en butée contre ledit siège d'insertion (33) et ledit siège de rétraction (35) ;
ledit corps de couvercle (12) dudit boîtier supérieur (10) a au moins une partie de sollicitation (123) au niveau d'une surface périphérique interne de celui-ci, ledit siège d'insertion (33) ayant au moins une partie de boucle (335) qui vient séparément en butée contre une partie d'arrêt (317) dudit corps principal (31) de manière à former une structure de limitation d'insertion (A) entre ledit siège d'insertion (33) et ledit corps principal (31) ;
dans lequel, lorsque ledit boîtier inférieur (20) est séparé dudit boîtier supérieur (10) et lorsque ledit boîtier supérieur (10) est enfoncé vers une surface cutanée, ladite au moins une partie de boucle (335) dudit siège d'insertion (33) est poussée par ladite au moins une partie de sollicitation (123) dudit boîtier supérieur (10) pour être déformée vers l'intérieur et pour être séparée de ladite partie d'arrêt (317) dudit corps principal (31) dudit ensemble corps principal (300), de sorte que ladite structure de limitation d'insertion (A) est affaissée ; et
dans lequel, après l'enfoncement dudit corps de couvercle (12), ladite structure de mise en prise de boîtier (124) dudit boîtier supérieur (10) se met en prise avec ladite structure de mise en prise de corps (315) dudit ensemble corps principal (300) de telle sorte que ledit boîtier supérieur (10) soit positionné par rapport audit ensemble corps principal (300), et ladite au moins une partie de sollicitation (123) dudit boîtier supérieur (10) est bloquée sous ledit corps principal (31) de telle sorte que ledit corps de couvercle (12) ne puisse pas être déplacé vers le haut.

2. Dispositif d'insertion selon la revendication 1, dans lequel ladite structure de mise en prise de corps (315) est disposée sur ledit corps principal (31) dudit ensemble de corps principal (300), ladite structure de mise en prise de corps (315) étant capable de se mettre en prise avec ladite structure de mise en prise de boîtier (124).

3. Dispositif d'insertion selon la revendication 1, comprenant en outre un élément de fixation (40), ledit élément de fixation (40) étant monté de manière coulissante sur ledit corps principal (31) dudit ensemble corps principal (300), ledit élément de fixation (40) interférant avec ledit corps de couvercle (12) et ledit boîtier inférieur (20) de manière à empêcher une opération d'insertion involontaire dudit dispositif d'insertion lorsque ledit boîtier inférieur (20) est accouplé audit boîtier supérieur (10).

4. Dispositif d'insertion selon la revendication 3, dans lequel ledit élément de fixation (40) a une partie de poussée (41) qui correspond en position audit boîtier inférieur (20), une partie de support (42) qui est opposée à ladite partie de poussée (41), une structure de crochet de fixation (43) qui est située entre ladite partie de poussée (41) et ladite partie de support (42), et une partie de came (44) qui est située entre ladite partie de poussée (41) et ladite partie de support (42).

5. Dispositif d'insertion selon la revendication 4, comprenant en outre un ensemble capteur (70) qui est couplé audit module d'insertion (30) et qui est supporté par ladite partie de support (42) dudit élément de fixation (40).

6. Dispositif d'insertion selon la revendication 5, comprenant en outre une base (50), ladite base (50) ayant des structures de crochet de base (54) qui correspondent à ladite structure de crochet de fixation (43) dudit élément de fixation (40), lesdites structures de crochet de base (54) se mettant en prise avec ladite structure de crochet de fixation (43) dudit élément de fixation (40) pour positionner ladite base (50) dans ledit dispositif d'insertion, ledit ensemble capteur (70) étant séparé dudit module d'insertion (30) et positionné sur ladite base (50) pendant l'opération d'insertion dudit dispositif d'insertion.

7. Dispositif d'insertion selon la revendication 5, comprenant en outre une aiguille d'insertion (36) qui est accouplée audit module d'insertion (30) et déplacée par celui-ci, ladite aiguille d'insertion (36) étant accouplée à un siège d'insertion auxiliaire (38), ledit ensemble capteur (70) étant monté de manière amovible sur ledit siège d'insertion auxiliaire (38).

8. Dispositif d'insertion selon la revendication 1, dans lequel ledit siège d'insertion (33) a une rainure de limitation (330), ledit couvercle principal (32) ayant un élément de limitation (323) qui se met en prise de manière amovible avec ladite rainure de limitation (330) dudit siège d'insertion (33), ledit siège d'insertion (33) ayant en outre une partie de positionnement de rétraction (334) qui limite les mouvements dudit siège de rétraction (35) par rapport audit siège d'insertion (33), ladite partie de positionnement de rétraction (334) dudit siège d'insertion (33) étant limitée par ledit élément de limitation (323) dudit couvercle principal (32), de manière à former une structure de limitation de rétraction (B) entre ledit siège de rétraction (35), ledit siège d'insertion (33) et ledit couvercle principal (32) dudit ensemble corps principal (300).

9. Dispositif d'insertion selon la revendication 8, dans lequel lorsque ledit siège d'insertion (33) dudit module d'insertion (30) se déplace vers le bas pour effectuer une opération d'insertion automatique, ledit élément de limitation (323) dudit couvercle principal (32) est séparé de ladite rainure de limitation (330), de sorte qu'une limitation structurelle entre ledit siège d'insertion (33) et ledit couvercle principal (32) est supprimée.

10. Dispositif d'insertion selon la revendication 9, dans lequel lorsque la limitation structurelle entre ledit siège d'insertion (33) et ledit couvercle principal (32) est supprimée, ladite partie de positionnement de rétraction (334) est autorisée à être déformée vers l'extérieur pour supprimer la structure de limitation de rétraction (B), de manière à supprimer une limitation structurelle entre ledit siège de rétraction (35) et ledit siège d'insertion (33).

11. Dispositif d'insertion selon la revendication 6, dans lequel ladite base (50) a une partie de montage (500) destinée à permettre audit ensemble de détection (70) d'être monté de manière amovible sur celle-ci, ladite partie de montage (500) de ladite base (50) comportant un évidement (501) qui permet audit ensemble de détection (70) d'être emmanché à force dans celui-ci, une couche adhésive (502) qui est disposée dans ledit évidement (501) destiné à permettre audit ensemble de détection (70) d'être collé sur celle-ci, et au moins un crochet élastique (503) qui est destiné à se mettre en prise avec ledit ensemble de détection (70).

12. Dispositif d'insertion selon la revendication 1, dans lequel ledit boîtier inférieur (20) est accouplé hermétiquement et séparément audit boîtier supérieur (10) de manière à former un espace hermétique à l'intérieur dudit dispositif d'insertion, ledit espace hermétique étant pourvu d'un absorbeur d'humidité.

13. Dispositif d'insertion selon la revendication 6, comprenant en outre une paroi d'amortissement (80) qui est disposée sur une face interne d'une partie inférieure dudit boîtier supérieur (10), ladite base (50) étant plus haute qu'un bord inférieur de ladite paroi d'amortissement (80) avant que ledit dispositif d'insertion ne soit actionné.
